(19) Europäisches Patentamt
European Patent Office
Office européen des brevets

(11) Publication number: 0 161 904 B1

(12) # EUROPEAN PATENT SPECIFICATION

(45) Date of publication of patent specification: 18.10.89

(21) Application number: 85303255.5

(22) Date of filing: 08.05.85

(51) Int. Cl.$^4$: C 07 D 307/16, C 07 D 333/24, A 61 K 31/34, A 61 K 31/38 // C07D319/08, C07D307/87, C07D307/12, C07D407/12, C07D309/12, C07D317/24, C07C35/18, C07D409/14, C07D409/04, C07F7/18

(54) **Tetrahydrofuranyl and tetrahydrothienyl substituted ethers.**

(30) Priority: 10.05.84 US 609054
17.12.84 US 682712

(43) Date of publication of application: 21.11.85 Bulletin 85/47

(45) Publication of the grant of the patent: 18.10.89 Bulletin 89/42

(84) Designated Contracting States: BE CH DE FR GB IT LI LU NL SE

(56) References cited:
EP-A-0 029 247

JOURNAL OF THE AMERICAN CHEMICAL SOCIETY, vol. 104, no. 6, 24th March 1982, pages 1621-1628, Gaston, US; C.H. LIN et al.: "10-Nor-9,11-secoprostaglandins. Synthesis, structure, and biology of endoperoxide analogues"

(73) Proprietor: E.R. Squibb & Sons, Inc.
Lawrenceville-Princeton Road
Princeton, N.J. 08540 (US)

(72) Inventor: Das, Jagabandhu
1-11 Fox Run Drive
Plainsboro New Jersey (US)

(74) Representative: Thomas, Roger Tamlyn et al
D. Young & Co. 10 Staple Inn
London WC1V 7RD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 161 904 B1

Courier Press, Leamington Spa, England.

**Description**

The present invention concerns tetrahydrofuranyl substituted ethers which are cardiovascular agents useful, for example, in the treatment of thrombolytic disease. These compounds have the general formula

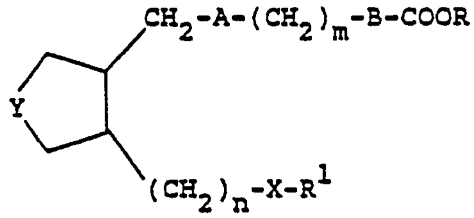

(I)

(including all stereoisomers thereof)

wherein A is $(CH_2)_2$, CH=CH or a single bond; m is 1 to 8; B is a single bond or CH=CH; R is H, lower alkyl or alkali metal; n is 1 to 4, X is O or

S
||
$(O)_{n'}$

wherein n′ is 0, 1 or 2; and $R^1$ is lower alkyl, arylalkyl, aryl, cycloalkyl, cycloalkylalkyl or lower alkenyl and Y is either oxygen or sulfur.

The term "lower alkyl" or "alkyl" as employed herein includes both straight and branched chain radicals of up to 12 carbons, preferably 1 to 8 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including a halo-substituent, such as F, Br, Cl or I or $CF_3$, an alkoxy substituent, an aryl substituent (for example,

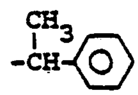 ),

an alkyl-aryl substituent, a haloaryl substituent, a cycloalkyl substituent or an alkylcycloalkyl substituent.

The term "cycloalkyl" includes saturated cyclic hydrocarbon groups containing 3 to 12 carbons, preferably 3 to 8 carbons, which include cyclopropyl, cyclobutyl, cyclopentyl, cyclohexyl, cycloheptyl, cyclooctyl, cyclodecyl and cyclododecyl, any of which groups may be substituted with 1 or 2 halogens, 1 or 2 lower alkyl groups and/or lower alkoxy groups.

The term "aryl" or "Ar" as employed herein refers to monocyclic or bicyclic aromatic groups containing from 6 to 10 carbons in the ring portion, such as phenyl, naphthyl, substituted phenyl or substituted naphthyl wherein the substituent on either the phenyl or naphthyl may be lower alkyl, halogen (Cl, Br or F), or lower alkoxy.

The term "lower alkenyl" or "alkenyl" includes straight or branched chain radicals of from 2 to 12 carbons, preferably 2 to 6 carbons in the normal chain, which includes one double bond in the normal chain, such as 2-propenyl, 3-butenyl, 2-butenyl, 1-pentenyl, 3-pentenyl, 2-hexenyl, 3-hexenyl, 2-heptenyl, 3-heptenyl, 4-heptenyl, 3-octenyl, 3-nonenyl, 4-decenyl, 3-undecenyl, 4-dodecenyl and the like.

The term "aralkyl", "aryl-alkyl" or "aryl-lower alkyl" as used herein refers to lower alkyl groups as discussed above having an aryl substituent, such as benzyl or methylbenzyl

( 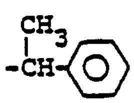 ).

The term "cycloalkylalkyl" as used herein refers to cycloalkyl groups as defined above linked to an alkyl group as defined above.

The term "lower alkoxy", "alkoxy" or "aralkoxy" includes any of the above lower alkyl, alkyl or aralkyl groups linked to an oxygen atom.

The term "halogen" or "halo" as used herein refers to chlorine, bromine, fluorine or iodine with chlorine being preferred.

The term "$(CH_2)_m$", $(CH_2)_{n'}$ and "$(CH_2)_2$" includes straight chain radicals having from 1 to 8 carbons and 1 to 4 carbons in the normal chain in the case of $(CH_2)_{m'}$ and $(CH_2)_{n'}$ respectively, and 2 carbons in the normal chain in the case of $(CH_2)_2$, which may in addition contain one or more lower alkyl substituents. Examples of $(CH_2)_{m'}$, $(CH_2)_n$ or $(CH_2)_2$ groups respectively, include

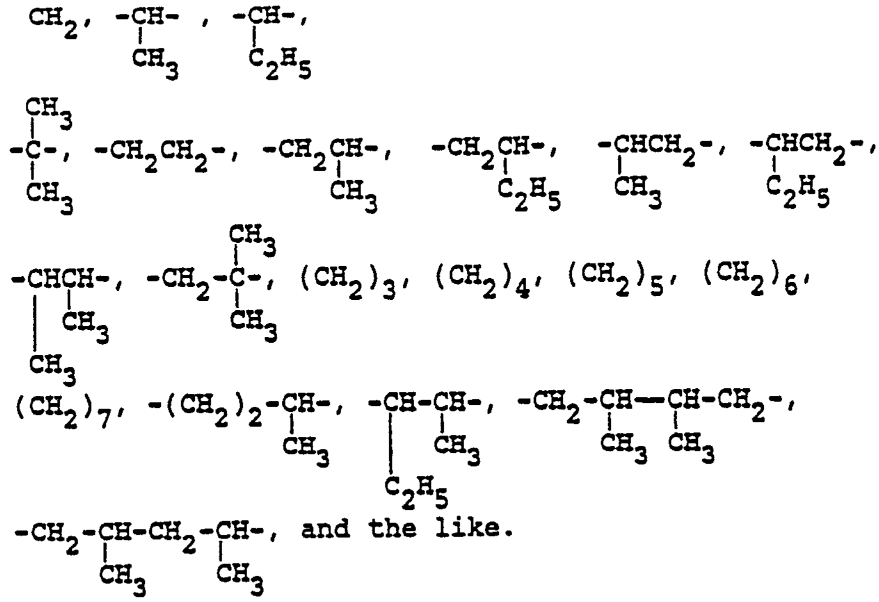

and the like.

Preferred are those compounds of formula I wherein A is —CH=CH— or —$(CH_2)_2$—, B is a single bond, m is 2 or 5, n is 1 or 2, R is hydrogen and $R^1$ is lower alkyl, phenyl, cycloalkyl or benzyl.

Preferred compounds include those where (a) A is CH=CH and B is a single bond, (b) X is O or S, (C) m is 3 to 5 and n is 1, (d) $R^1$ is H, (e) B is a single bond, n is 1, A is CH=CH, X is O or S, n is 1, m is 3 to 5, R is H and $R^1$ is lower alkyl, and (d) $R^1$ is butyl, pentyl, hexyl or heptyl including all isomers thereof.

It should be noted that Journal of the American Chemical Society, vol. 104, no. 6, 24th March 1982, pages 1621—1628, Gaston, US; C.H. Lin et al: "10-Nor-9,11-secoprostaglandins. Synthesis, structure, and biology of endoperoxide analogues" discloses compounds conforming to formula I as regards the nucleus and the first side chain wherein A is —CH=CH—, m is 3, B is a single bond but wherein the second side chain is —$CH=CH_2$—$C(OR_2)$—$(CH_2)_4$—$CH_3$. In one such compound $R_1=CH_3$ and $R_2=COCH_3$ and in another $R_1=R_2=H$.

Compounds of formula I of the invention of the cis series may be prepared using aldehyde II as the starting material

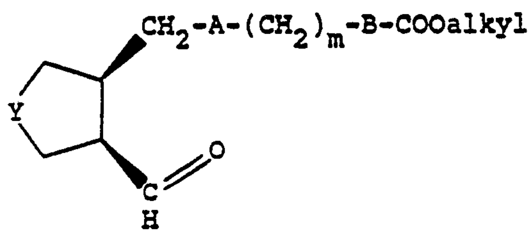

II

Thus, to prepare compounds of formula I wherein X is O, B is a single bond, A is CH=CH or $(CH_2)_2$ and n is 1, aldehyde II is reduced by treating II with a reducing agent such as sodium borohydride, or sodium cyanoborohydride in an inert organic solvent, such as methanol, to form alcohol III

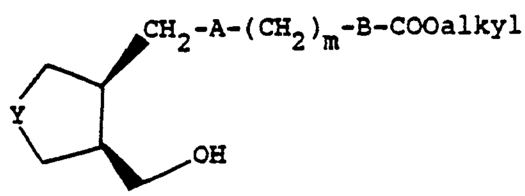

III

Alcohol III is then made to undergo ether formation by reacting III with a compound of the structure IV

$$Z—R^1 \quad \text{IV}$$

wherein Z is Cl, Br, I, —$OSO_2CH_3$ or

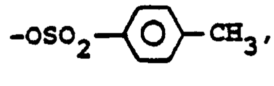

in the presence of potassium hydroxide or other strong base and an inert organic solvent such as xylene, tetrahydrofuran (THF), dimethylsulfoxide (DMSO) or dimethylformamide (DMF) employing a molar ratio of III:IV of within the range of from about 0.2:1 to about 0.1:1, to form V

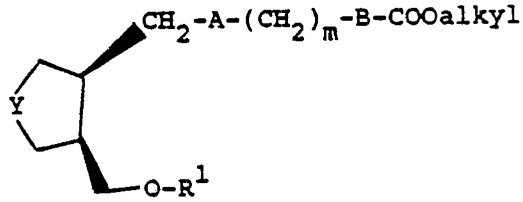

V

Where in formula IV, Z is Br or Cl, a phase transfer etherification is employed in which case THF is used as the solvent and a transfer reagent such as $(C_4H_9)_4$ $NHSO_4$ or $(C_6H_5CH_2)(CH_3)_2NHSO_4$ is employed.

The ester V may then be hydrolyzed by treatment with a strong base such as lithium hydroxide, potassium carbonate or sodium hydroxide in the presence of an inert organic solvent such as tetrahydrofuran to form the corresponding alkali metal salt which is treated with a strong acid, such as HCl, to form the cis compounds of the invention

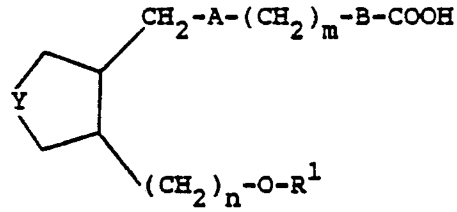

VA

Compounds of formula I of the invention of the trans series wherein X is O, and B is a single bond, may be prepared using aldehyde IIA

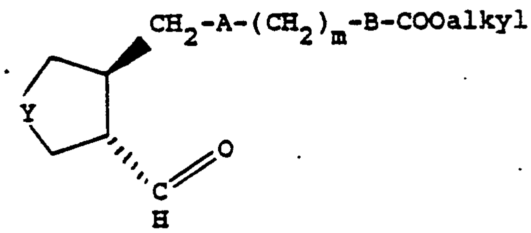

IIA

which itself is prepared from aldehyde II by simply subjecting II to an epimerization reaction wherein II is reacted with sodium methoxide in the presence of methanol to form aldehyde IIA.

Aldehyde IIA is then reduced by treatment with sodium borohydride or sodium cyanoborohydride in the presence of an inert organic solvent such as methanol to form alcohol VI

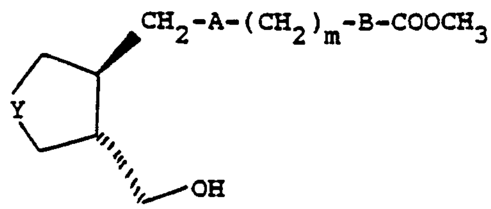

VI

which is then etherified by reaction with a mesylate, tosylate or halide IV

$$Z—R^1 \qquad IV$$

as described above with respect to the cis series to form ester VII

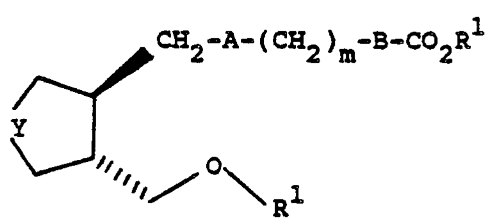

VII

Ester VII is then hydrolyzed by treatment with a strong base such as lithium hydroxide, potassium carbonate or sodium hydroxide in the presence of an inert organic solvent such as tetrahydrofuran, to form the corresponding alkali metal salt which is treated with a strong acid, such as HCl, to form the trans compounds of the invention VIII

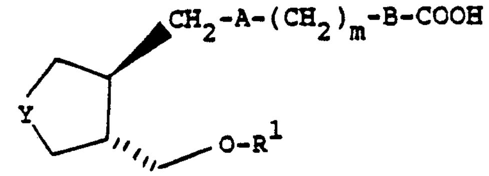

VIII

Compounds of the invention wherein X is S may be prepared from hydroxymethyl compound III or VI. Thus, where A is —CH=CH— and B is a single bond, compound III or VI is subjected to a tosylation reaction, for example, by reacting III or VI with tosyl chloride in pyridine to form tosylate IX

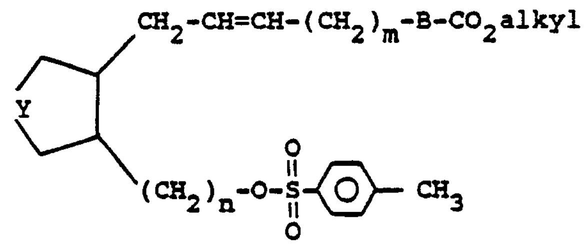

IX

To form the tosylate IXA (where A is $(CH_2)_2$, compound III or VI is reduced, for example, with hydrogen over a palladium on carbon catalyst, to form hydroxymethyl compound IIIA or VIA (where A is $(CH_2)_2$)

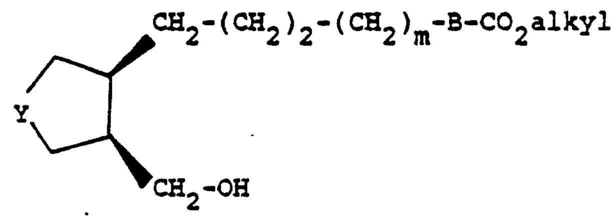

IIIA

or

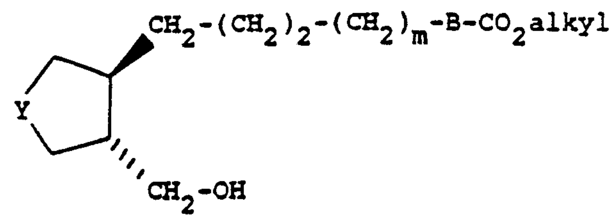

VIA

Compound IIIA or VIA is subjected to a tosylation reaction to form tosylate IXA (where A is $(CH_2)_2$)

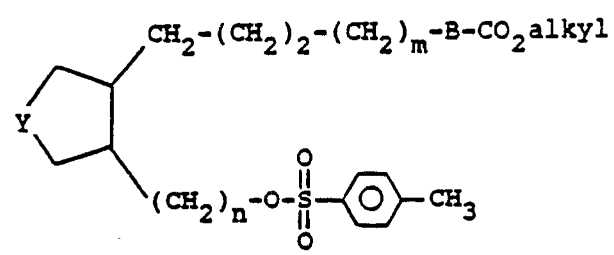

IXA

Thereafter, tosylate IX or IXA is reacted with a thiol of the structure

$$R^1SH \qquad X$$

employing a molar ratio of IX or IXA: thiol of within the range of from about 0.8:1 to about 1:1, in a solvent such as tetrahydrofuran and in the presence of potassium t-butoxide to form the sulfide XI or XIA

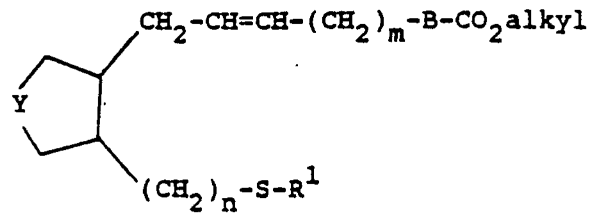

XI

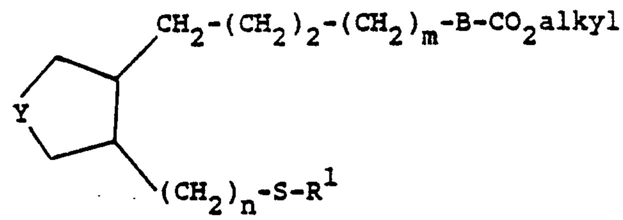

XIA

To form the sulfinyl and/or sulfonyl analogs (where n'=1), sulfide derivative XI or XIA is subjected to oxidation, for example, by reacting same with sodium periodate, in the presence of methanol and tetrahydrofuran, to form the sulfinyl derivative XII or XIIA and the sulfonyl derivative XIII or IIIA. The sulfinyl and sulfonyl derivatives may be separated by chromatography or other conventional separation procedures.

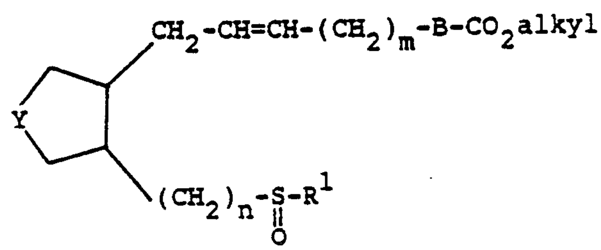

XII

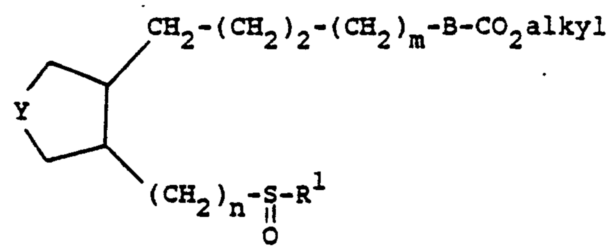

XIIA

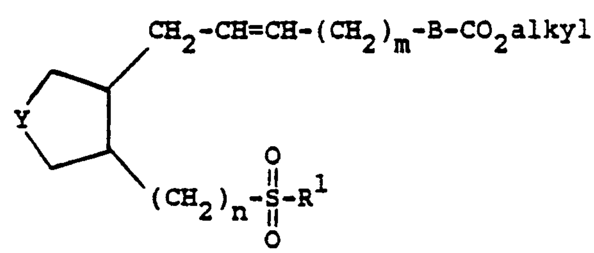

XIII

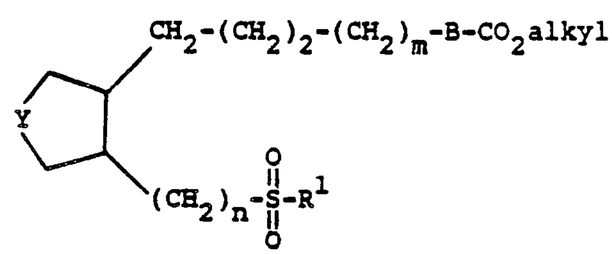

XIIIA

Compounds of Formula I wherein n is 2 to 4, may be prepared starting with the starting lower alkyl ester containing the hydroxymethyl group, that is, compound III or VI which is used to form the aldehyde II' (where A is —CH=CH—) or II'' (where A is —$(CH_2)_2$).

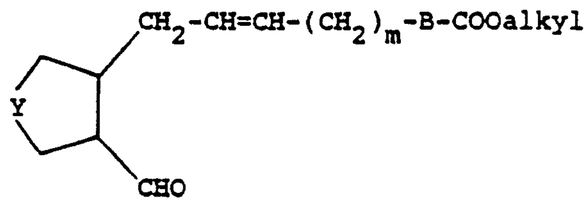

II'

(includes cis and/or trans)

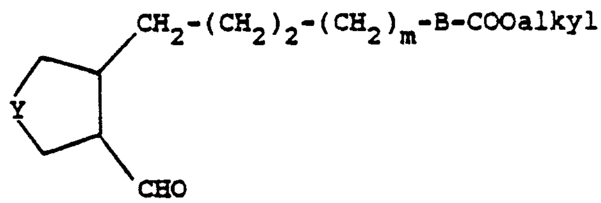

II''

Thus, to form aldehyde II' where A is —CH=CH—, compound III or VI is subjected to a Collins oxidation, for example, by reacting III or VI with chromium trioxide in pyridine. To form the aldehyde II'' (where A is $(CH_2)_2$), compound III or VI is reduced, for example, with hydrogen over a palladium on carbon catalyst, to form hydroxymethyl compound IIIA or VIA as described above and compound IIIA or VIA is subjected to a Collins oxidation to form aldehyde II'' (where A is $(CH_2)_2$ including all isomers).

The aldehyde II' or II'' is used to prepare aldehyde XV (where n is 2—4) by carrying out a homologation sequence, such as a Wittig reaction with $(C_6H_5)_3P$=CHOMe followed by hydrolysis, (n-1) times, as shown below.

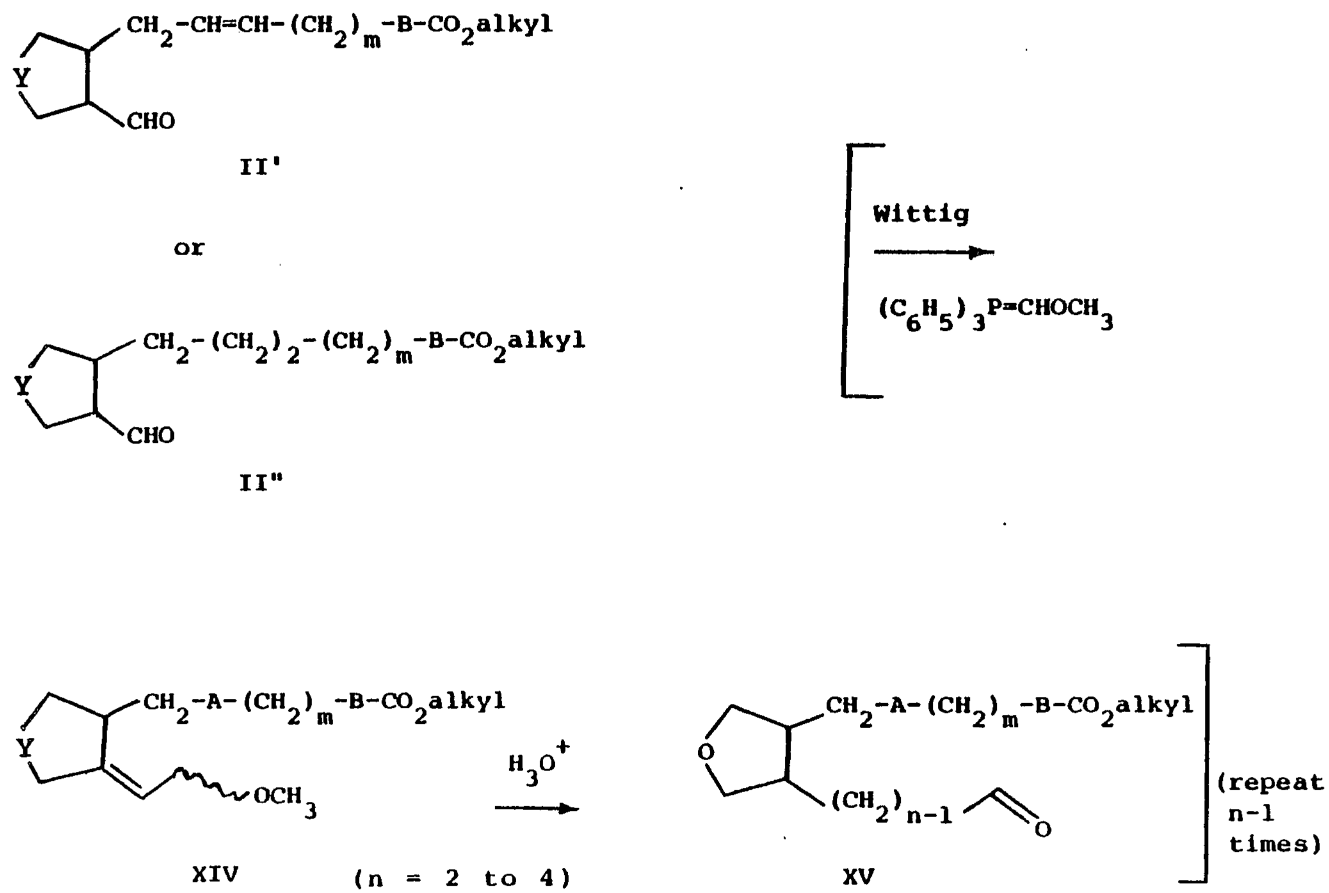


II'

or

II"

XIV

XV

The aldehyde XV (where n is 2—4) is thus carried on to compounds of this invention where n is 2—4, that is

IA
or
IB

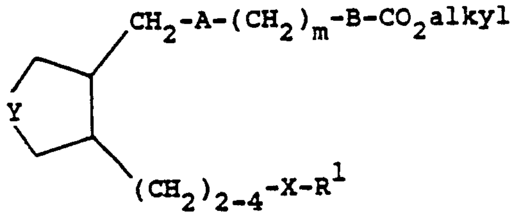

(IA where A is $-CH{=}CH-$)
(IB where A is $(CH_2)_2$)

by reducing aldehyde XV employing a reducing agent such as sodium borohydride or sodium cyanoborohydride in a solvent such as methanol to form the alcohol ester XVI

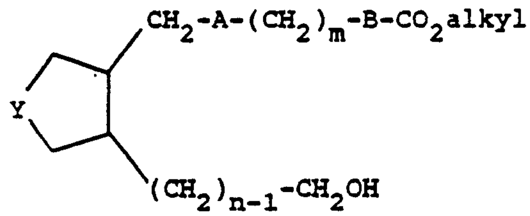

XVI

which is subjected to a tosylation/mesylation reaction as described above to form the corresponding tosylate/mesylate which in turn is subjected to ether formation by reaction with

$$Z—R^1 \qquad \text{IV}$$

or

$$R^1SH \qquad A$$

as described above to form ether IC or thioether ID

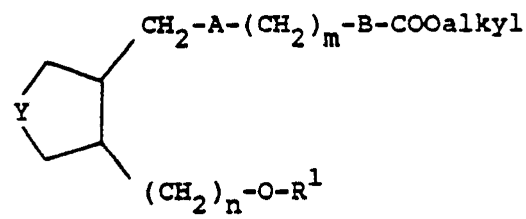

IC

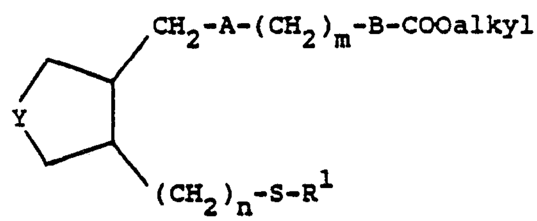

ID

The sulfinyl derivatives (where n is 2 to 4) and sulfonyl derivatives (where n is 2 to 4) are prepared by subjecting thioether ID (n'=0) to an oxidation reaction as described above to form a mixture of sulfinyl (n'=1) and sulfonyl derivatives (n'=2).

The above sulfinyl and sulfonyl derivatives may be separated by chromatography or other conventional separation procedures.

The various esters XI, XIA, XII, XIIA, XIII, XIIIA, IA, IB, IC, and ID can be converted to the free acid, that is, to

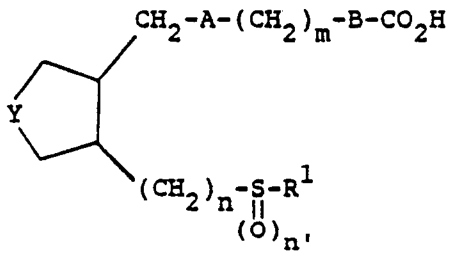

IE

by treatment of the esters with an alkali metal hydroxide, such as lithium or sodium hydroxide to form the alkali metal salt, followed by neutralization with an acid, such as dilute hydrochloric acid or oxalic acid to form the acid IE.

Compounds of the invention wherein B is —CH=CH— and A is CH=CH or $(CH_2)_2$ may be prepared as follows. The tetrahydrofuranyl ether or thio ether of the structure XVII

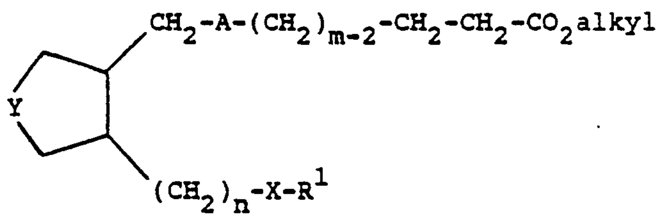

XVII

is subjected to phenylselenylation by reacting XVII with lithium temperatures of from about 0 to about −78°C in the presence of an inert organic solvent such as tetrahydrofuran, dimethoxy ethane or ether; thereafter a solution of diphenyl-diselenide in an inert organic solvent as described above is added and the reaction is maintained at reduced temperatures as described above to form the selenophenyl ester XVIII

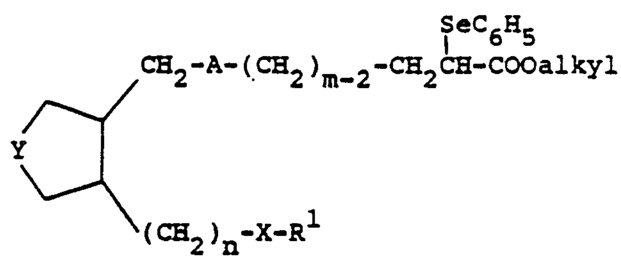

XVIII

The selenophenyl ester XVIII is next hydrolyzed by reaction with a strong base such as LiOH, $K_2CO_3$ or NaOH and then treated with strong acid such as HCl as described hereinbefore to form acid XIX

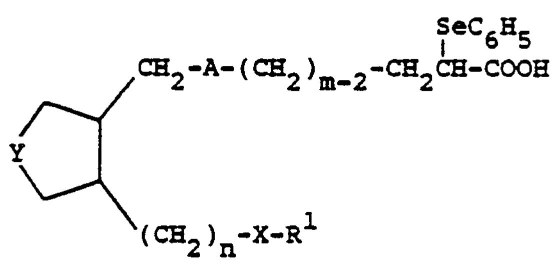

XIX

The selenophenyl acid XIX is then made to undergo a selenoxide elimination reaction wherein the selenophenyl acid is treated with hydrogen peroxide in an inert organic solvent such as tetrahydrofuran to form acid XX

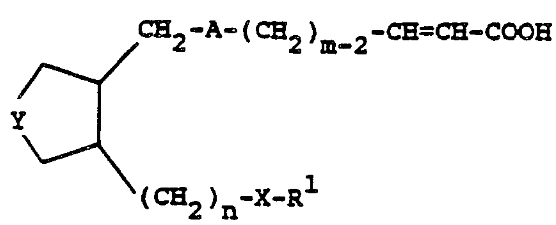

XX

The tris(hydroxymethyl)aminomethane salt of any of the acids of formula I of the present invention is formed by reacting a solution of such acid in an inert solvent such as methanol with tris(hydroxymethyl)-aminomethane and thereafter the solvent is removed by evaporation to leave the desired salt.

The aldehyde intermediate II

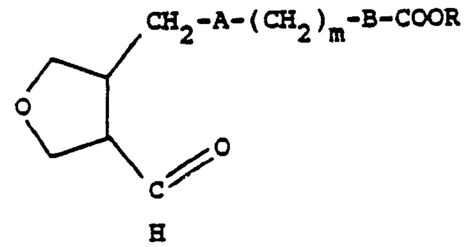

II

wherein A is —CH=CH may be prepared as follows.

1-Trimethylsilyloxy-1,3-butadiene *A* in an inert organic solvent such as methylene chloride, ether or tetrahydrofuran is made to react with maleic anhydride *B* in a Diels-Alder reaction to form the anhydride *C*

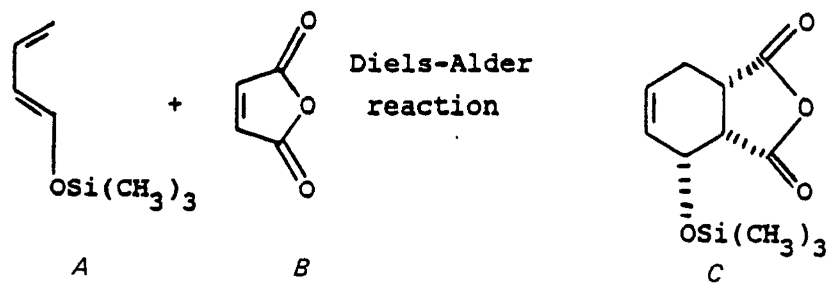


*A* *B* *C*

The anhydride *C* is reduced, for example, by treatment with a reducing agent such as lithium aluminum hydride, in the presence of an inert organic solvent such as tetrahydrofuran to form triol *D*

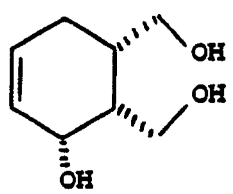

*D*

The triol *D* is then made to undergo acetonide formation by reacting *D* with p-toluene sulfonic acid in an inert organic solvent such as acetone, or with 2,2-dimethoxy propane or 2-methoxypropene in methylene chloride and under an inert atmosphere to form acetonide *E*

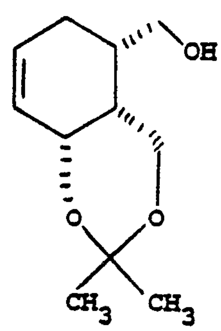

*E*

Acetonide *E* is then tosylated by reacting *D* in a solution of methylene chloride and weak organic base such as pyridine, with p-toluenesulfonyl chloride to form the tosylate *F*

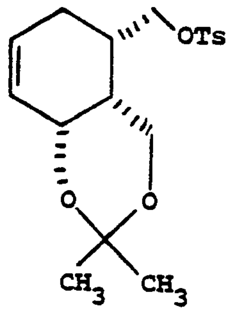

F

Tosylate *F* is then hydrolyzed by treatment with strong acid, such as HCl, oxalic acid or amberlyst resin/methanol in the presence of an inert organic solvent such as tetrahydrofuran to form alcohol *G*

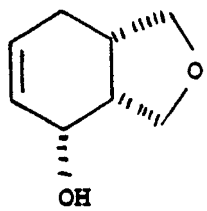

G

Next, the alcohol *G* is benzylated by reacting *G* with benzylbromide in the presence of sodium hydride and an inert organic solvent such as dimethylformamide to form benzylether *H*

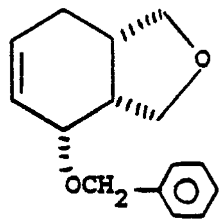

H

which is then made to undergo osmylation by reacting *H* with osmium tetroxide in the presence of N-methylmorpholine-N-oxide and appropriate inert organic solvent such as tetrahydrofuran to form diol *J*

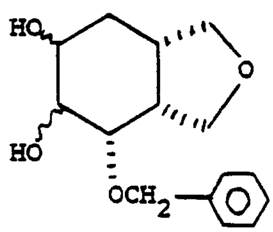

J

The diol *J* is next subjected to periodate cleavage by reacting diol *J* in an alcohol solvent such as methanol with sodium metaperiodate to form dialdehyde *K*

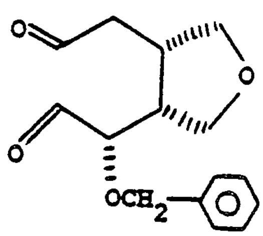

K

The dialdehyde *K* is then reduced by treatment with lithium aluminum hydride or other reducing agent such as sodium borohydride or lithium borohydride in the presence of an inert organic solvent such as methanol or tetrahydrofuran, to form the diol *L*

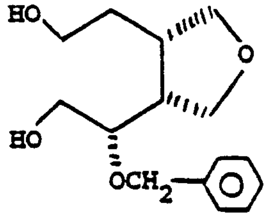

L

which is then made to undergo hydrogenolysis by treatment of *L* with hydrogen in the presence of a palladium over carbon catalyst in ethyl acetate and glacial acetic acid, to form triol *M*

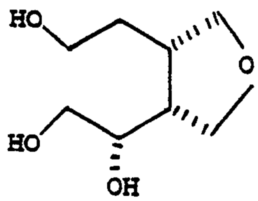

M

The triol *M* is next subjected to acetonide formation by reacting *M* with p-toluenesulfonic acid in the presence of an inert organic solvent such as acetone, to form the alcohol *N*

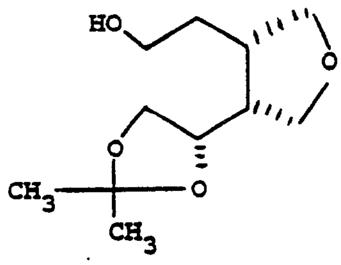

N

which is oxidized by reacting with pyridinium chlorochromate in the presence of an inert organic solvent such as methylene chloride or with chromium trioxide in pyridine, to form aldehyde *O*

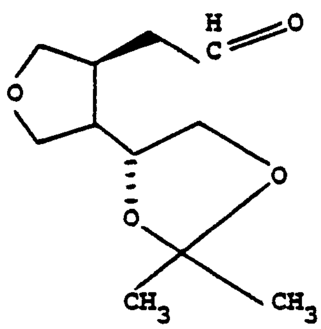

O

Aldehyde *O* is next subjected to a Wittig reaction wherein a mixture of triphenylphosphonium compound *P*

$$(C_6H_5)_3P—A—(CH_2)_m—B—COOH \cdot Br \qquad P$$

such as (4-carboxybutyl)-triphenylphosphonium bromide salt in tetrahydrofuran and potassium t-amylate in toluene is reacted with aldehyde *O* to form acid *Q*

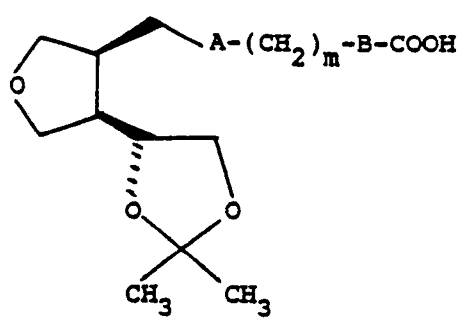

Q

which is then dissolved in ether and reacted with a diazoalkane such as diazomethane to form ester *R*

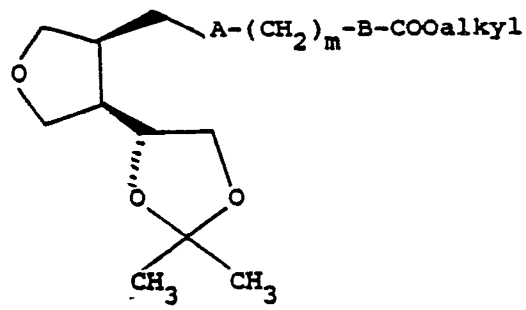

*R*

Ester *R* is then made to undergo acetal exchange by reacting *R* in methanol with p-toluene sulfonic acid to form diol *S*

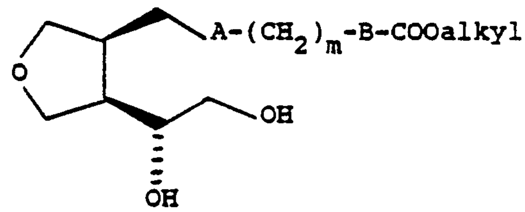

*S*

which is then subjected to periodate cleavage by reacting *S* in methanol with sodium metaperiodate to form aldehyde IIA

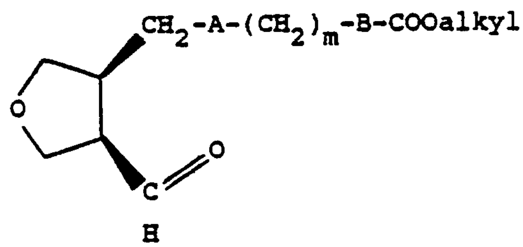

IIA

(wherein A is CH=CH)

The aldehyde intermediate II

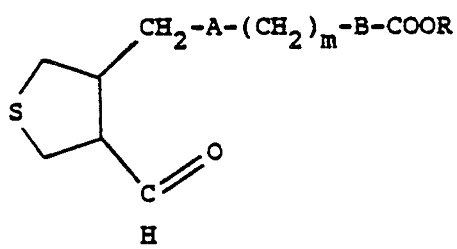

II

wherein A is —CH=CH may be prepared as follows.

1-Trimethylsilyloxy-1,3-butadiene *A* in an inert organic solvent such as methylene chloride, ether or tetrahydrofuran is made to react with maleic anhydride *B* in a Diels-Alder reaction to form the anhydride *C*

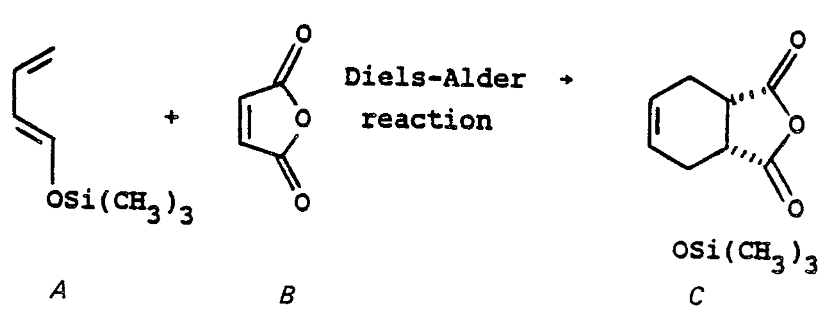


*A* *B* *C*

The anhydride *C* is treated with concentrated hydrochloric acid in the presence of an inert organic solvent such as tetrahydrofuran to form the desilylated adduct *D'*

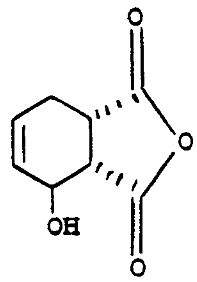

*D'*

which is then reacted with dihydropyran in the presence of dry methylene chloride and p-toluenesulfonic acid to form the tetrahydropyranyl ether *E'*

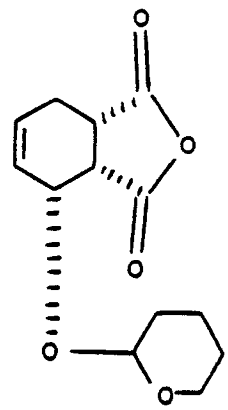

*E'*

The ether *E'* is reduced, for example, by treatment with a reducing agent such as lithium aluminum hydride, in the presence of an inert organic solvent such as tetrahydrofuran to form diol *F'*

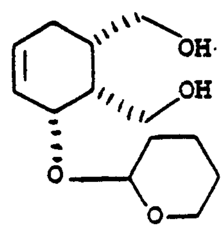

*F'*

The diol *F'* is then reacted with dimethylamino pyridine and methyl chloroformate in the presence of an inert organic solvent such as methylene chloride and a base such as pyridine to form bis-methylcarbonate *G'*

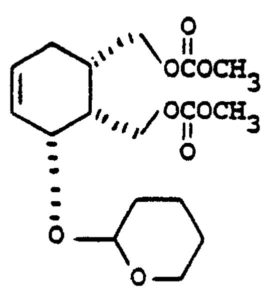

*G'*

which is then made to undergo osmylation by reacting *G'* with osmium tetroxide in the presence of N-methylmorpholine-N-oxide and appropriate inert organic solvent such as tetrahydrofuran to form diol *H'*

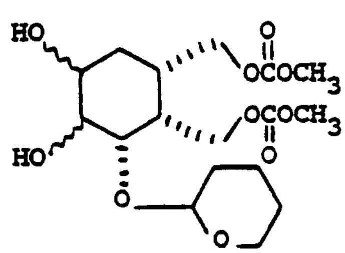

*H'*

The diol *H'* is next subjected to periodate cleavage by reacting diol *H'* in an alcohol solvent such as methanol with sodium metaperiodate to form dialdehyde *J'*

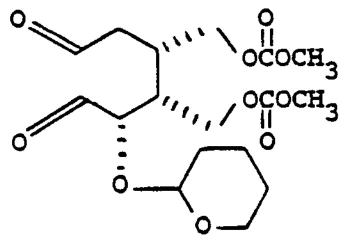

*J'*

The dialdehyde *J'* is then reduced by treatment with a reducing agent such as sodium borohydride or lithium borohydride in the presence of an inert organic solvent such as methanol or tetrahydrofuran, to form the diol *K'*

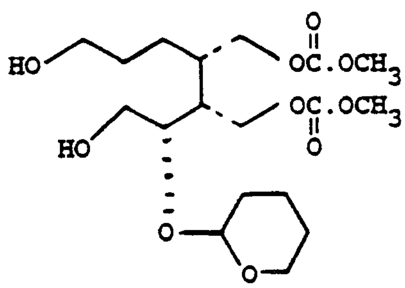

*K'*

which is then subjected to acetonide formation by treating K' with dry Amberlyst-15 acid resin in the presence of methanol and acetone to form the alcohol *L'*

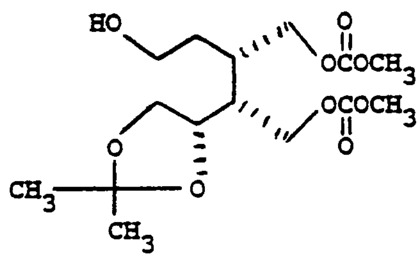

*L'*

which is treated with p-toluenesulfonic acid and dihydropyran in the presence of an inert organic solvent such as methylene chloride, to form tetrahydropyranyl ether *M'*

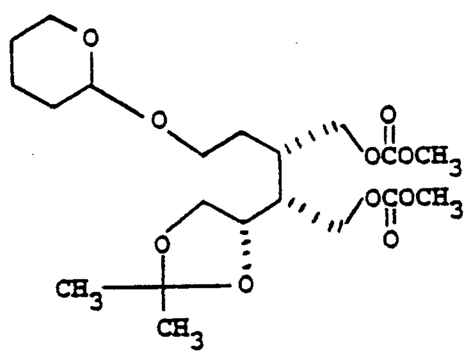

*M'*

which is then reduced to the diol *N'* by treating *M'* with lithium aluminum hydride in the presence of an inert solvent such as tetrahydrofuran

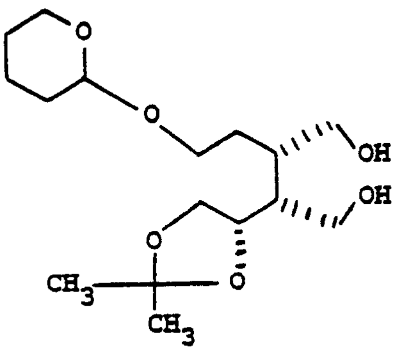

N'

The diol *N'* is then reacted with methanesulfonyl chloride in the presence of an organic solvent such as pyridine to form the bis-mesylate *O'*

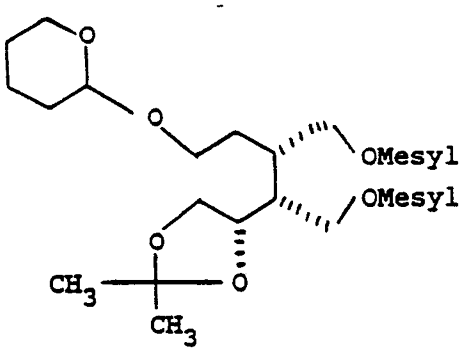

O'

The bis-mesylate *O'* in dimethylsulfoxide or methanol is treated with sodium sulfide nonahydrate in dimethyl sulfoxide or methanol to form the tetrahydrothiophane *P'*

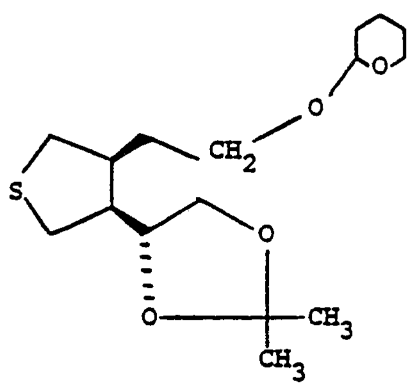

P'

which is treated with Amberlyst-15 resin in the presence of methanol and acetone to form the alcohol *Q'*

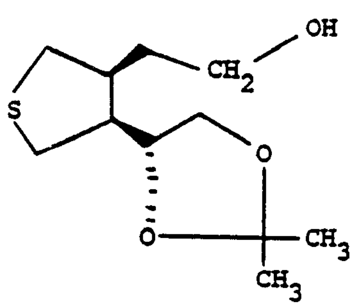

Q'

Alcohol *Q'* is then treated with dimethylsulfoxide in the presence of oxalyl chloride and methylene chloride and then with triethylamine to form the aldehyde *R'*

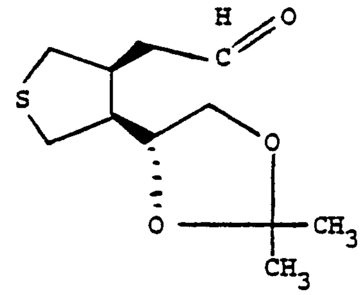

R'

Aldehyde *R'* is next subjected to a Wittig reaction wherein a mixture of triphenylphosphonium compound *P*

$$(C_6H_5)_3P—A—(CH_2)_m—B—COOH \cdot Br \qquad P$$

such as (4-carboxybutyl)-triphenylphosphonium bromide salt in tetrahydrofuran and potassium t-amylate in toluene is reacted with aldehyde *R'* to form acid *T*

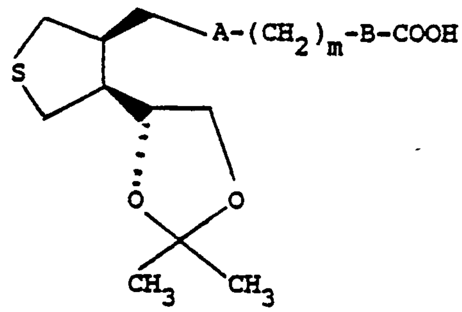

T

which is then dissolved in ether and reacted with a diazoalkane such as diazomethane to form ester *U*

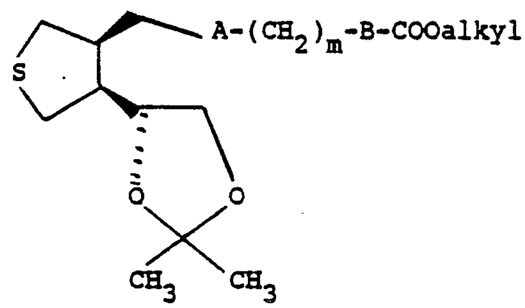

U

Ester *R'* is then made to undergo acetal exchange by reacting *R'* in methanol with p-toluene sulfonic acid to form diol *V*

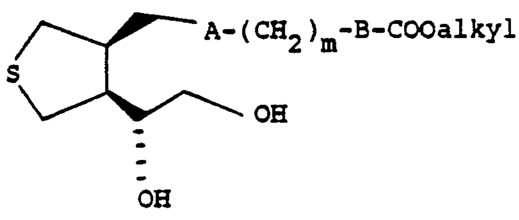

V

which is then subjected to periodate cleavage by reacting *V* in methanol with sodium metaperiodate to form aldehyde IIA

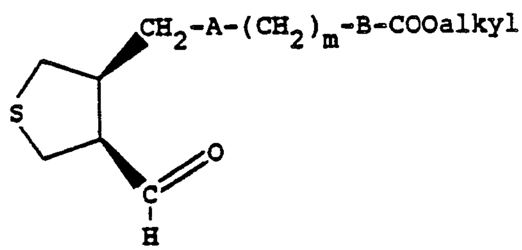

IIA

(wherein A is CH=CH)

The intermediate aldehyde of formula II wherein A is $-(CH_2)_2-$ are prepared by reducing compound *S* by treatment with hydrogen in the presence of palladium on charcoal to form compound *S'*

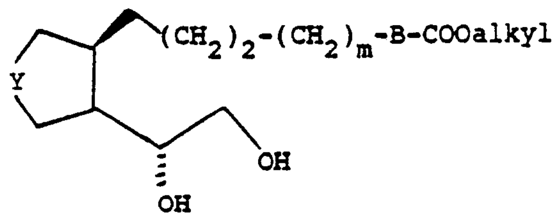

*S'*

which is subjected to periodate cleavage as described above to form aldehyde *IIAA*

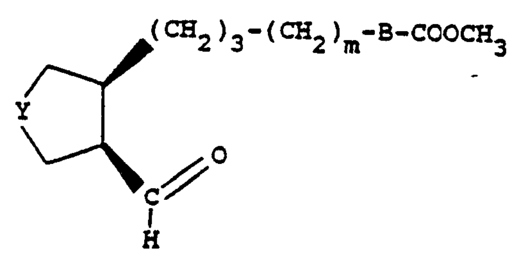

*IIAA*

which may then be hydrolyzed to the corresponding acid XIX by treatment with alkali metal hydroxide and then HCl as described hereinbefore.

The compounds of this invention have three centers of asymmetry as indicated by the asterisks in formula I. However, it will be apparent that each of the formulae set out above which do not include asterisks still represent all of the possible stereoisomers thereof. All of the various stereoisomeric forms are within the scope of the invention.

The various stereoisomeric forms of the compounds of the invention, namely, cis and trans forms and stereoisomeric pairs may be prepared as shown in the working Examples which follow. Examples of such stereoisomers are set out below.

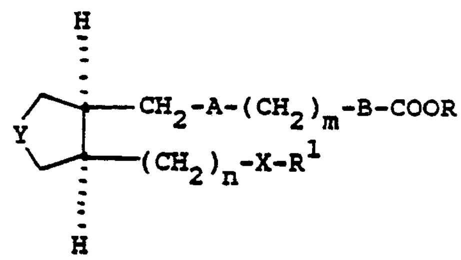

Ia

(cis)

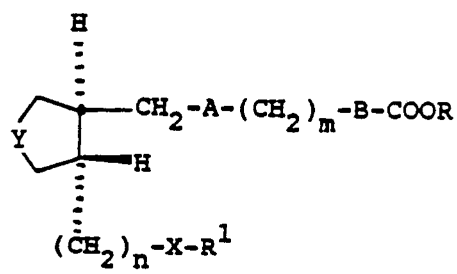

Ib

(trans)

The compounds of this invention are cardiovascular agents useful as platelet aggregation inhibitors, such as inhibiting arachidonic acid-induced platelet aggregation (e.g., for treatment of thrombolytic disease, such as coronary or cerebral thromboses) and in inhibiting bronco-constriction as induced by asthma. They are also selective thromboxane $A_2$ receptor antagonists and synthetase inhibitors, e.g., having a vasodilatory effect for treatment of myocardial ischemic disease, such as angina pectoris. The compounds of the invention are also arachidonic acid cyclooxygenase inhibitors. In addition, the compounds of the invention are useful as analgesic agents in the manner of aspirin and indomethacin as indicated by reaction thresholds to pressure in edematous hindpaws [Ref: Winter et al, J. Pharmacol, Exp. Ther. 150:165, 1965] and as antiinflammatory agents in mammals, as indicated by carrageenin-induced edema in the rat [Ref: Winter et al., J. Pharmacol., Exp. Ther. 141:369, 1963]. They may be used to decrease joint swelling, tenderness, pain and stiffness in conditions such as rheumatoid arthritis.

The compounds of the invention can be administered orally or parenterally to various mammalian species known to be subject to such maladies, e.g., cats, dogs, and the like in an effective amount within the dosage range of about 1 to 100 mg/kg, preferably about 1 to 50 mg/kg and especially about 2 to 25 mg/kg on a regimen in single or 2 to 4 divided daily doses.

The compounds of the invention may also be administered topically to any of the above mammalian species in amounts of from about 0.1 to 10 mg/kg in single or 2 to 4 divided daily doses.

The active substance can be utilized in a composition such as tablet, capsule, solution or suspension containing about 5 to about 500 mg per unit of dosage of a compound or mixture of compounds of formula I. They may be compounded in conventional matter with a physiologially acceptable vehicle or carrier, excipient, binder, preservative, stabilizer, flavor, etc. as called for by accepted pharmaceutical practice. Also as indicated in the discussion above, certain members additionally serve as intermediates for other members of the group.

The compounds of this invention may also be administered topically to threat peripheral vascular diseases and as such may be formulated as a cream or ointment.

The following Examples represent preferred embodiments of the invention. Unless otherwise indicated, all temperatures are expressed in degrees Centigrade.

Example 1

[3α(Z),4α]-7-[4-[(Hexyloxy)methyl]tetrahydro-3-furanyl]-5-heptanoic acid

A. [3α(Z),4α]-7-[Tetrahydro-4-formyl-3-furanyl]-5-heptanoic acid, methyl ester

(1) (1α,2β,3β)-1-Trimethylsilyloxy-cyclohex-5-ene, 2,3-dicarboxylic acid anhydride

To a solution of 28.4 g of 1-trimethylsilyloxy-1,3-butadiene (0.2 mole) in 200 ml of $CH_2Cl_2$ at 25°C was added 19.6 g of maleic anhydride (0.2 mole). The mixture was stirred at 25°C for 24 hours then concentrated. The residue was purified on a LPS—1 silica gel column, eluting with 5% EtOAc/hexanes (3 liters) and 20% EtOAc/hexanes (4 liters) to give 26.0 g of title anhydride as a light yelow oil.

(2) (1α,2β,3β)-1-Hydroxy-cyclohex-5-ene, 2,3-dimethanol

To a suspension of 8.0 g of lithium aluminum hydride (210.5 mmole, 2 eq.) in 200 ml of dry THF at 0°C was added slowly, a solution of 25 g of title A (1) anhydride (104 mmole) in 150 ml of dry THF. The reaction was stirred at reflux for 4 hours and at 25°C for 18 hours, then cooled to 0°C and a saturated solution of $Na_2SO_4$ was added dropwise until no more white precipitates formed. It was then filtered. The white precipitates were washed thoroughly with THF, then stirred with 500 ml of 10% acetonitrile in ethylacetate for 30 minutes and filtered. The combined filtrate was concentrated to give a viscous oil which was purified on a LPS—1 silica gel column, eluting with 50% EtOAc/hexanes and 5% methanol/EtOAc to give 14.98 g of title triol as a clear oil.

(3) (1α,9β,10β)-1-Hydroxymethyl-6,6-dimethyl-3,4-dehydro-5,7-dioxa-octalin

To a solution of 14.98 g of title A (2) triol (95 mmole) in 150 ml of dry acetone was added 30 g of dried 4A molecular sieves and 1 g of p-toluenesulfonic acid (5 mmole, 5 mole %). After stirring at 25°C under an argon atmosphere for 18 hours, the reaction mixture was neutralized with solid sodium bicarbonate and filtered. The filtrate was concentrated to give an oil which was purified on a LPS—1 silica gel column, eluting with 5% EtOAc/hexanes and 10% EtOAc/hexanes to give 15.58 g of the title acetonide.

(4) (1α,9β,10β)-1-Toluenesulfonyloxymethyl-6,6-dimethyl-3,4-dehydro-5,7-dioxa-octalin

To a solution of 6 g of title A (3) acetonide (30 mmole) in 40 ml of dry methylene chloride and 20 ml of pyridine (150 mmole, 5 eq.) was added 7.63 g of p-toluenesulfonylchloride (40 mmole, 1.3 eq.). After stirring at 25°C for 24 hours, the reaction mixture was diluted with ether and washed with water, 1*N* hydrochloric acid and brine. The aqueous layer was back-extracted with ether. The combined organic layer was dried over anhydrous $MgSO_4$ and concentrated to give title tosylate in the form of an oil which was used directly in the next reaction.

(5) (4α,8β,9β)-4-Hydroxy-1,3,4,7,8,9-hexahydro-isobenzofuran

To a solution of crude title A (4) tosylate (6.30 mmole) in 40 ml of dry THF and 10 ml of $H_2O$ was added 20 ml of a 1*N* aqueous HCl solution. After stirring for 6 hours at 25°C, the reaction was neutralized with solid $NaHCO_3$ and diluted with methylene chloride. The layers were separated. The aqueous layer was extracted with methylene chloride. The combined organic layer was dried over anhydrous $MgSO_4$ and concentrated to give an oil which was purified on an LPS—1 silica gel column, eluting with 5—10% EtOAc/hexanes to give 3.85 g of title alcohol.

(6) (4α,8β,9β)-4-Benzyloxy-1,3,4,7,8,9-hexahydro-isobenzofuran

To a slurry of 1.44 g of prewashed sodium hydride (50% dispersion in mineral oil, 27.0 mmole, 1.6 eq.) in 20 ml of dry DMF at 0°C was added a solution of 3.85 g title A (5) alcohol (27.0 mmole) in 10 ml DMF. The mixture was stirred at 25°C for 15 minutes, cooled to 0°C and then 4.3 g of benzylbromide (27.0 mmole, 1.0 eq.) was added. After stirring for 30 minutes at 25°C, the reaction mixture was poured into 300 ml of a saturated aqueous ammonium chloride solution and extracted with three 100 ml of water, dried over

anhydrous $MgSO_4$ and concentrated. The residue was purified on an LPS—1 silica gel column, eluting with 10% EtOAc/hexanes to give 4.5 g of title benzylether as a yellow oil.

(7) (4α,8β,9β)-4-Benzyloxy-5,6-dihydroxy-octahydro-isobenzofuran

To 1.6 g of title A (6) benzylether (6.95 mmole) in 70 ml of dry THF at 25°C was added 1.17 g of N-methylmorpholine-N-oxide (8.34 mmole, 1.2 eq.) followed by dropwise addition of water until a homogeneous solution was obtained. To the resulting solution at 25°C was added 353 μmole of a 5% solution of osmium tetroxide in ether (67.5 μmole, 1%). After stirring at 25°C for 2 hours, 30 ml of a saturated aqueous sodium bisulfite solution was added to the mixture which was stirred at 25°C for 30 minutes and extracted with three 100 ml portions of $CH_2Cl_2$. The combined organic layer was washed with 50 ml of 1*N* HCl solution, 50 ml of $H_2O$, dried over anhydrous $MgSO_4$ and concentrated to give 1.6 g title diol as a light brown solid. This was used without purification.

(8) [3α,4α(1S)]-2-Tetrahydro-4-(1-benzyloxy-1-formylmethyl)-3-furanyl]acetaldehyde

To 1.6 g title A (7) diol (6.06 mmole) in 40 ml of methanol at 25°C was added a solution of 1.48 g of sodium metaperiodate (6.0 mmole, 1.1 eq.) in 15 ml of water. After stirring at 25°C for 1 hour, the reaction mixture was extracted with three 50 ml portions of $CH_2Cl_2$. The organic layer was dried over anhydrous $MgSO_4$ and concentrated to give 1.7 g title dialdehyde as a yellow oil. This was used without purification.

(9) [3α,4α(1S)]-2-Tetrahydro-4-(1-benzyloxy-1-hydroxymethylmethyl)-3-furanyl]ethanol

To a slurry of 460 mg of lithium aluminum anhydride (12.1 mmole, 4 eq.) in 50 ml of dry THF at 0°C was added slowly a solution of 1.7 g crude title A (8) dialdehyde (*ca* 6.0 mmole) in 10 ml of dry THF. After stirring at 0°C for 20 minutes, a saturated aqueous sodium sulfate solution was added dropwise until no more precipitates formed. The mixture was diluted with 300 ml of $CH_2Cl_2$ and stirred with anhydrous $MgSO_4$ for 30 minutes then filtered. The filtrate was concentrated to give 1.6 g title A(9) diol as a clear oil.

(10) [3α,4α(1S)]-2-Tetrahydro-4-(1-hydroxy-1-hydroxymethylmethyl)-3-furanyl]ethanol

A mixture of 1.6 title A (9) diol, 1.6 g of 10% palladium over carbon in 80 ml of EtOAc and 4 ml of glacial acetic acid was shaken in a Parr bottle under 50 lb of hydrogen pressure at 25°C for 24 hours. The mixture was then filtered through a bed of Celite. The filtrate was concentrated to give title A (10) triol as a clear oil. this oil was used without purification.

(11) (3α,4α)-2-[Tetrahydro-4-(4,4-dimethyl-3,5-dioxa-cyclopentyl)-3-furanyl]ethanol

To title A (10) triol (*ca*. 6.0 mmole) in 20 ml of dry acetone was added 113 mg of p-toluenesulfonic acid (0.6 mmole, 10%) and 1.0 g of molecular sieves type 4Å. After stirring at 25°C for 4 hours, the reaction mixture was neutralized by addition of 80 mg solid sodium bicarbonate and filtered. The filtrate was concentrated to give a crude oil which was purified on a silica gel column, eluting with 50% EtOAc/hexanes (2 liters) and 3% $MeOH/CH_2Cl_2$ (1 liter) to give 1.0 g of title A (11) alcohol as a clear oil.

(12) (3α,4α)-2-[Tetrahydro-4-(4,4-dimethyl-3,5-dioxa-cyclopentyl)-3-furanyl]acetaldehyde

To 300 mg of title A (11) alcohol (1.4 mmole) in 10 ml of $CH_2Cl_2$ was added 1.0 g of Celite, followed by 595 mg of pyridinium chlorochromate (2.8 mmole, 2 eq.). After stirring for 2 hours at 25°C, the reaction mixture was diluted with 100 ml of ether and filtered through a bed of florosil. The filtrate was concentrated to give title A (12) aldehyde as a clear oil. This was used directly in the next reaction.

(13) [3α(Z),4α]-7-[Tetrahydro-4-(4,4-dimethyl-3,5-dioxa-cyclopentyl)-3-furanyl]-5-heptenoic acid and

(14) [3α(Z),4α]-7-[Tetrahydro-4-(4,4-dimethyl-3,5-dioxa-cyclopentyl)-3-furanyl]-5-heptenoic acid

To 927 mg of (4-carboxybutyl)-triphenylphosphonium bromide salt (2.1 mmole, 1.5 eq.) in 5 ml of dry THF at 0°C was added dropwise 2.7 ml of a 1.43*M* solution of potassium t-amylate in toluene (3.9 mmole, 2.8 eq.). The mixture was stirred at 25°C for 2 hours, cooled to 0°C and a solution of title A (12) aldehyde in 5 ml of THF (*ca*. 1.4 mmole) was added dropwise. After stirring at 25°C for 1 hour, the reaction was quenched with glacial acetic acid and poured into 300 ml of brine and extracted with three 50 ml portions of EtOAc. The combined organic layer was concentrated. The residue was diluted with 50 ml of a saturated sodium bicarbonate solution, then extracted with three 50 ml portions of EtOAc. The aqueous layer was acidified to pH 5 with glacial acetic acid and extracted with four 50 ml portions of $CH_2Cl_2$. The organic layer was dried over anhydrous $MgSO_4$ and concentrated to give title A (13) acid as a oil. This oil was dissolved in ether and methanol and treated with excess $CH_2N_2$ in ether to give 400 mg of a yellow oil after concentration. Purification was done on a silica gel column, eluting with 30% EtOAc/hexanes to give 210 mg of title A (14) ester as a yellow oil.

(15) [3α(Z),4α(1S)]-7-[Tetrahydro-4-[(1-hydroxy-1-hydroxymethyl)methyl]-3-furanyl]-5-heptenoic acid, methyl ester

To 180 mg of title A (14) ester (0.57 mmole) in 2 ml of methanol was added 5.4 mg of p-toluene sulfonic acid (28.8 μm, 5%). The mixture was stirred at 25°C for 4 hours, then concentrated. The residue was

dissolved in 2 ml of fresh methanol and stirred at 25°C for 18 hours, then concentrated. The residue was diluted with 30 ml of ether and filtered through a bed of silica gel. The filtrate was concentrated to give 127 mg of title diol as a clear oil.

(16) [3α(Z),4α]-7-[Tetrahydro-4-formyl-3-furanyl]-5-heptenoic acid, methyl ester

To a solution of 127 mg of title A (15) diol (0.40 mmole) in 5 ml of methanol at 25°C was added a solution of 107 mg of sodium metaperiodate in 1 ml of $H_2O$. The mixture was stirred at 25°C for 30 minutes, then extracted with three 10 ml portions of $CH_2Cl_2$. The organic layer was dried over anhydrous $MgSO_4$ and concentrated to give title aldehyde as a clear oil.

B. [3α(Z),4α]-7-[(4-Hydroxymethyl)tetrahydro-3-furanyl]-5-heptenoic acid, methylester

To 136.8 mg of title A aldehyde (0.57 mmole) in 2 ml of methanol at 0°C was added 21.7 mg of sodium borohydride (0.57 mmole, 4 eq.). After stirring for 15 minutes, the mixture was poured into 100 ml of a saturated ammonium chloride solution and extracted with three 30 ml portions of ether. The organic layer was dried over anhydrous $MgSO_4$ and concentrated to give 128 mg of title alcohol. This was used without purification.

C. [3α(Z),4α]-7-[4-[(Hexyloxy)methyl]tetrahydro-3-furanyl]-5-heptenoic acid

A solution of 280 mg of potassium hydroxide (5 mmole, 10 eq.) in 20 ml of dry xylene was distilled off until about 10 ml of the solution remained. To this solution was added a solution of 120 mg of title B alcohol (0.5 mmole) in 20 ml of dry xylene. The mixture was heated to reflux and 10 ml of xylene was again distilled off. To the cooled remaining solution was added a solution of 430 mg of hexylmesylate (2.5 mmole, 5 eq.) in 10 ml of dry xylene. The resulting mixture was refluxed for 3 hours, cooled to 25°C, diluted with 100 ml of $H_2O$, and extracted with three 50 ml portions of ether. The combined ethereal extract was washed with two 30 ml portions of $H_2O$, dried over anhydrous $MgSO_4$ and concentrated.

The residue was purified on a CC—7 silica gel column, eluting with a gradient of pentane/ether. The product thus collected was kept under high vacuum for 2 days to yield 37 mg of title compound as a clear oil.

TLC: Silica gel; 7% $MeOH/CH_2Cl_2$; $R_f$~0.45.

| | | |
|---|---|---|
| Anal Calcd for $C_{18}H_{32}O_4$ 0.13 $H_2O$: | C, 68.69; | H, 10.33 |
| Found: | C, 68.69; | H, 10.36 |

Example 2

[3α(Z),4β]-7-[4-[(Hexyloxy)methyl]tetrahydro-3-furanyl]-5-heptenoic acid, hexyl ester

A. [3α(Z),4β]-7-[Tetrahydro-4-formyl-3-furanyl]-5-heptenoic acid, methyl ester

To 140 mg [3α(Z),4α]-7-[tetrahydro-4-formyl-3-furanyl]-5-heptenoic acid, methyl ester (prepared as described in Example 1, Part A (16) (0.58 mmole) in 2 ml of methanol was added 3.15 mg of sodium methoxide (58 μmole, 10%). After stirring at 25°C for 2 hours, the reaction mixture was poured into 50 ml of a saturated aqueous ammonium chloride solution and extracted with three 10 ml portions of ether. The organic layer was washed with 10 ml of $H_2O$ and dried over anhydrous $MgSO_4$ and concentrated to give 130 mg of title aldehyde as an oil. This was used without purification.

B. [3α(Z),4β]-7-[Tetrahydro-4-hydroxymethyl-3-furanyl]-5-heptenoic acid, methyl ester

To 43 mg of title A aldehyde (0.18 mmole) in 1 ml of methanol at 0°C was added 6.8 mg of sodium borohydride (0.18 mmole, 4 eq.). After stirring at 0°C for 10 minutes, the mixture was poured into 20 ml of a saturated $NH_4Cl$ solution and extracted with three 10 ml portions of ether. The combined ethereal extract was dried over anhydrous $MgSO_4$ and concentrated to give 44 mg of title alcohol as an oil.

C. [3α(Z),4β]-7-[4-[(Hexyloxy)methyl]tetrahydro-3-furanyl]-5-heptenoic acid, hexyl ester

To 100 mg of powdered potassium hydroxide (1.8 mmole, 10 eq.) in 20 ml of dry xylene was added a solution of 44 mg of title B alcohol (0.18 mmole) in 20 ml of dry xylene. The mixture was heated to reflux and *ca.* 20 ml of xylene was distilled off.

To the cooled remaining solution was added a solution of 309 mg of hexyl mesylate (1.8 mmole, 10 eq.) and the mixture was heated at reflux for 3 hours. The mixture was cooled to 25°C, diluted with 100 ml of ether and washed with two 20 ml portions of water. The organic layer was dried over anhydrous $MgSO_4$ and concentrated to give an oil which was purified on a silica gel column, eluting with 10% EtOAc/hexanes to yield 45 mg of title ester as a yellow oil.

Example 3

[3α(Z),4β]-7-[4-[(Hexyloxy)methyl]tetrahydro-3-furanyl]-5-heptenoic acid

To 45 mg of Example 2 title C ester (0.14 mmole) in 6 ml of THF was aded 1.6 ml of 1*M* lithium hydroxide solution. The mixture was stirred at 25°C for 4 days and then concentrated. The residue was diluted with 10 ml of $H_2O$ and acidified with a saturated oxalic acid solution to pH 3 and extracted with three 10 ml portions of ether. The combined ethereal extract was washed with two 10 ml portions of water, dried over anhydrous $MgSO_4$ and concentrated.

The residue was purified on a CC—7 silica gel column, eluting with a gradient of pentane/ether. The product was kept under high vacuum for 2 days to yield 28 mg of title compound as an oil.

TLC: silica gel; 5% $MeOH/CH_2Cl_2$; $R_f$~0.4.

Anal Calcd for $C_{18}H_{32}O_4$: C, 69.14; H, 10.32

Found: C, 68.94; H, 10.39

Example 4

[3α(Z),4α]-7-[4-[([Hexylthio)methyl]tetrahydro-3-furanyl]-5-heptenoic acid, methyl ester

A. [3α(Z),4α]-7-[(4-p-toluenesulfonyloxymethyl)tetrahydro-3-furanyl]-5-heptenoic acid, methyl ester

To 75 mg of [3α(Z),4α]-7-[tetrahydro-4-hydroxymethyl-3-furanyl]-5-heptenoic acid, methyl ester (prepared as described in Example 1 Part B (0.35 mmole) in 1 ml of pyridine at 25°C was added 90 mg of p-toluenesulfonyl chloride (0.39 mmole, 1.1 equiv.). The mixture was stirred at 25°C for 1.5 hours, then diluted with 30 ml of ether. The ethereal solution was washed with two 10 ml portions of a saturated cupric sulfate solution, 20 ml of $H_2O$, then dried over anhydrous $MgSO_4$ and concentrated. Purification was done on a silica gel column, eluting with 25% EtOAc/hexane to yield 70 mg of title compound as an oil.

B. [3α(Z),4α]-7-[4-[(Hexylthio)methyl]tetrahydro-3-furanyl]-5-heptenoic acid

To 50 ml of hexanethiol (0.35 mmol, 2 equiv.) in 1 ml of dry THF was added 24.7 mg of potassium t-butoxide (0.22 mmole, 1.3 equiv.). After stirring at 25°C for 30 minutes, a solution of 70 mg of title A tosylate in 1 ml of THF was added and the mixture was heated at reflux for 1 hour. The cooled mixture was diluted with 30 ml of ether and washed with two 10 ml portions of saturated $NaHCO_3$ and two 10 ml portions of $H_2O$. The organic layer was dried over anhydrous $MgSO_4$ and concentrated.

The residue was purified on a silica gel column, eluting with 30% EtOAc/hexane to give 37 mg of title ester as an oil.

Example 5

[3α(Z),4α]-7-[4-[(Hexylthio)methyl]tetrahydro-3-furanyl]-5-heptenoic acid

To a solution of 37 mg of Example 4 title B ester in 4 ml of THF, saturated with argon, was added at 25°C 1 ml of a 1 *M* lithium hydroxide solution. The mixture was stirred at 25°C under an argon atmosphere for 20 hours and then concentrated. The residue was diluted with 5 ml of $H_2O$, acidified to pH 3 with a saturated oxalic acid solution, extracted with three 20 ml portions of ether. The ethereal solution was washed with two 15 ml portions of $H_2O$, dried over anhydrous $MgSO_4$ and concentrated.

The product was kept under high vacuum for 2 days to yield 30 mg of title acid as an oil.

TC: silica gel; 5% $MeOH/CH_2Cl_2$; $R_f$~0.35

Anal Calcd for $C_{18}H_{32}O_3S$: C, 65.81; H, 9.76

Found: C, 65.57; H, 9.82

Example 6

[3α(Z),4α]-7-[4-[(Phenyloxy)methyl]tetrahydro-3-furanyl]-5-heptenoic acid

(a) Phenol (1 mmole) is added to a solution of 262 mg triphenylphosphine (1 mmole), diethylazodicarboxylate (1 mmole) and Example 1, Part B (1 mmole) alcohol in 25 ml of dry THF and is stirred at 23°C for 48 hours. The reaction mixture is concentrated *in vacuo* and the residue is triturated with ether. Filtration, concentration of the filtrate under reduced pressure and finally chromatogrpahy of the residue on a silica gel column gives [3α(Z),4α]-7-[4-[(phenyloxy)methyl]tetrahydro-3-furanyl]-5-heptenoic acid, methyl ester.

(b) Following the procedure set out in Example 3, the ester from part (a) is converted to the title acid.

Example 7

[3α(Z),4α]-7-[4-[(Benzyloxy)methyl]tetrahydro-3-furanyl]-5-heptenoic acid

Following the procedure of Example 1 except substituting benzyl mesylate for hexyl mesylate, the title compound is obtained.

Example 8

[3α(Z),4α]-7-[4-[(Cyclohexyloxy)methyl]tetrahydro-3-furanyl]-5-heptenoic acid

Following the procedure of Example 1 except substituting cyclohexyl mesylate for hexyl mesylate, the title compound is obtained.

Example 9

[3α(Z),4α]-7-[4-[(2-Pentenyloxy)methyl]tetrahydro-3-furanyl]-5-heptenoic acid

Following the procedure of Example 1 except substituting 2-pentenyl-1-mesylate for hexyl mesylate, the title compound is obtained.

Example 10

[3α(Z),4α]-7-[4-[(Cyclopentylmethyloxy)methyl]tetrahydro-3-furanyl]-5-heptenoic acid

Following the procedure of Example 1 except substituting cyclopentylmethyl mesylate for hexyl

mesylate, the title compound is obtained.

Example 11

[3α(Z),4β]-7-[4-[(Heptyloxy)methyl]tetrahydro-3-furanyl]-5-heptenoic acid

Following the procedure of Examples 2 and 3 except substituting heptyl mesylate for hexyl mesylate, the title compound is obtained.

Example 12

[3α(Z),4β]-7-[4-[(Phenethyloxy)methyl]tetrahydro-3-furanyl]-5-heptenoic acid

Following the procedure of Examples 2 and 3 except substituting phenethyl mesylate for hexyl mesylate, the title compound is obtained.

Example 13

[3α(Z),4β]-7-[4-[(Cyclopentyloxy)methyl]tetrahydro-3-furanyl]-5-heptenoic acid

Following the procedure of Examples 2 and 3 except substituting cyclopentyl mesylate for hexyl mesylate, the title compound is obtained.

Example 14

[3α(Z),4β]-7-[4-[(Cyclohexyloxy)methyl]tetrahydro-3-furanyl]-5-heptenoic acid

Following the procedure of Examples 2 and 3 except substituting cyclopentyl mesylate for hexyl mesylate, the title compound is obtained.

Example 15

[3α(Z),4β]-7-[4-[(3-Butenyloxy)methyl]tetrahydro-3-furanyl]-5-heptenoic acid

Following the procedure of Examples 2 and 3 except substituting 3-butenyl mesylate for hexyl mesylate, the title compound is obtained.

Example 16

[3α(Z),4α]-7-[4-[(2-Hexenylthio)methyl]tetrahydro-3-furanyl]-5-heptenoic acid

Following the procedure of Examples 4 and 5 except substituting 2-hexenyl-1-thiol for hexanethiol, the title compound is obtained.

Example 17

[3α(Z),4α]-7-[4-[(Propylthio)methyl]tetrahydro-3-furanyl]-5-heptenoic acid

Following the procedure of Examples 4 and 5 except substituting propylthiol for hexanethiol, the title compound is obtained.

Example 18

[3α(Z),4α]-7-[4-[(Cycloheptylthio)methyl]tetrahydro-3-furanyl]-5-heptenoic acid

Following the procedure of Examples 4 and 5 except substituting the cycloheptylmercaptan for hexanethiol, the title compound is obtained.

Example 19

[3α(Z),4α]-7-[4-[(Benzylthio)methyl]tetrahydro-3-furanyl]-5-heptenoic acid

Following the procedure of Examples 4 and 5 except substituting benzylmercaptan for hexanethiol, the title compound is obtained.

Example 20

[3α(Z),4α]-7-[4-[(Phenylthio)methyl]tetrahydro-3-furanyl]-5-heptenoic acid

Following the procedure of Examples 4 and 5 except substituting phenylmercaptan for hexanethiol, the title compound is obtained.

Example 21

(3α,4α)-7-[4-[(Hexyloxy)methyl]tetrahydro-3-furanyl]heptanoic acid

A. [3α,4α(1S)]-7-[Tetrahydro-4-[(1-hydroxymethyl)methyl]-3-furanyl]heptanoic acid, methyl ester

A mixture of 600 mg of Example 1 Part A (15) diol, 100 mg of a 10% palladium over carbon in 80 ml of EtOAc and 4 ml of glacial acetic acid is shaken in a Parr bottle under 50 lb of hydrogen pressure at 25°C for 24 hours. The mixture is then filtered through a bed of Celite. The filtrate is concentrated to give title A diol.

B. (3α,4α)-7-[Tetrahydro-(1-formyl)-3-furanyl]heptanoic acid, methyl ester

To a solution of 600 mg of title A diol (1.9 mmole) in 5 ml methanol at 25°C is added a solution of 490 mg of Na m-periodate in 1 ml $H_2O$. The mixture is stirred at 25°C for 30 minutes, then extracted with 3—10 ml portions of $CH_2Cl_2$. the organic layer is dried over anhydrous $MgSO_4$ and concentrated to give title aldehyde.

C. [3α,4α]-7-[4-[(Hexyloxy)methyl]tetrahydro-3-furanyl]heptanoic acid

Following the procedure of Example 1 Parts B and C except substituting the above Part B aldehyde for the Example 1 Part A (16) aldehyde, the title acid is obtained.

Example 22

(3α,4α)-7-[4-[(Benzyloxy)methyl]tetrahydro-3-furanyl]heptanoic acid

Following the procedure of Example 21 except substituting benzyl mesylate for hexyl mesylate, the title compound is obtained.

Example 23

(3α,4α)-7-[4-[(Phenyloxy)methyl]tetrahydro-3-furanyl]heptanoic acid

A. (3α,4α)-7-[4-(Hydroxy)tetrahydro-3-furanyl]heptanoic acid, methyl ester

Following the procedure of Example 1 Part B except substituting Example 21, Part B aldehyde for Example 1 Part A aldehyde, the title alcohol is obtained.

B. (3α,4α)-7-[4-[(Phenyloxy)methyl]tetrahydro-3-furanyl]heptanoic acid

Following the procedure of Example 6 except substituting the above title A alcohol for Example 1 Part B alcohol, the title compound is obtained.

Example 24

(3α,4α)-7-[4-[(Cyclohexyloxy)methyl]tetrahydro-3-furanyl]heptanoic acid

Following the procedure of Example 21 except substituting cyclohexyl mesylate for hexyl mesylate, the title compound is obtained.

Example 25

(3α,4α)-7-[4-[(2-Butenyloxy)methyl]tetrahydro-3-furanyl]heptanoic acid

Following the procedure of Example 21 except substituting 2-butenyl-1-mesylate for hexyl mesylate, the title compound is obtained.

Example 26

(3α,4β)-7-[4-[(Benzyloxy)methyl]tetrahydro-3-furanyl]heptanoic acid

Following the procedure of Example 21 except substituting the Example 2 Part A aldehyde for the Example 1 Part A (16) aldehyde and substituting benzyl mesylate for hexyl mesylate, the title compound is obtained.

Example 27

(3α,4β)-7-[4-[(Phenyloxy)methyl]tetrahydro-3-furanyl]heptanoic acid

A. (3α,4β)-7-[4-Formyl-tetrahydro-3-furanyl]heptanoic acid, methyl ester

Following the procedure of Example 2 Part B except substituting Example 21 Part B aldehyde for Example 2 Part A aldehyde, the title aldehyde is obtained.

B. (3α,4β)-7-[4-[(Phenyloxy)methyl]tetrahydro-3-furanyl]heptanoic acid

Following the procedure of Example 23 except substituting the above Part A aldehyde for Example 21 Part B aldehyde in Example 23 Part A, the title acid is obtained.

Example 28

(3α,4β)-7-[4-[(Cyclopropyloxy)methyl]tetrahydro-3-furanyl]heptanoic acid

Following the procedure of Example 21 except substituting the Example 2 Part A aldehyde for the Example 1 Part A (16) aldehyde and substituting cyclopropyl mesylate for hexyl mesylate, the title compound is obtained.

Example 29

(3α,4β)-7-[4-[(Cyclopentylethyloxy)methyl]tetrahydro-3-furanyl]heptanoic acid

Following the procedure of Example 21 except substituting the Example 2 Part A aldehyde for the Example 1 Part A (16) aldehyde and substituting cyclopentylethyl mesylate for hexyl mesylate, the title compound is obtained.

Example 30

(3α,4β)-7-[4-[(3-Hexenyloxy)methyl]tetrahydro-3-furanyl]heptanoic acid

Following the procedure of Example 21 except substituting the Example 2 Part A aldehyde for the Example 1 Part A (16) aldehyde and substituting 3-hexenyl-1-mesylate for hexyl mesylate, the title compound is obtained.

Example 31

(3α,4α)-7-[4-[(Octylthio)methyl]tetrahydro-3-furanyl]heptanoic acid

Following the procedure of Example 21 Parts A and B and Examples 4 and 5 except substituting the Example 21 Part B aldehyde in Examples 4 and 5 for the Example 1 Parts A (16) aldehyde and substituting octylthiol for hexanethiol, the title compound is obtained.

Example 32

(3α,4α)-7-[4-[(Phenylthio)methyl]tetrahydro-3-furanyl]heptanoic acid

Following the procedure of Example 21 Parts A and B and Examples 4 and 5, substituting the Example 21 Part B aldehyde for the Example 1 Part A (16) aldehyde in Examples 4 and 5 and substituting phenylmercaptan for hexanethiol, the title compound is obtained.

Example 33

(3α,4α)-7-[4-[(Benzylthio)methyl]tetrahydro-3-furanyl]heptanoic acid

Following the procedure of Example 21 Parts A and B and Examples 4 and 5, substituting the Example 21 and Part B aldehyde for the Example 1 Part A (16) aldehyde in Examples 4 and 5 and substituting benzylmercaptan for hexanethiol, the title compound is obtained.

Example 34

(3α,4α)-7-[4-[(Cyclopentylthio)methyl]tetrahydro-3-furanyl]heptanoic acid

Following the procedure of Example 21 Parts A and B and Examples 4 and 5, substituting the Example 21 and Part B aldehyde for the Example 1 Part A (16) aldehyde in Examples 4 and 5 and substituting cyclopentylmercaptan for hexanethiol, the title compound is obtained.

Example 35

(3α,4α)-7-[4-[(Cyclohexylmethylthio)methyl]tetrahydro-3-furanyl]heptanoic acid

Following the procedure of Example 21 Parts A and B and Examples 4 and 5, substituting the Example 21 and Part B aldehyde for the Example 1 Part A (16) aldehyde in Examples 4 and 5 and substituting cyclohexylmethylmercaptan for hexanethiol, the title compound is obtained.

Example 36

(3α,4α)-7-[4-[(2-Octenylthio)methyl]tetrahydro-3-furanyl]heptanoic acid

Following the procedure of Example 21 Parts A and B and Examples 4 and 5, substituting the Example 21 and Part B aldehyde for the Example 1 Part A (16) aldehyde in Examples 4 and 5 and substituting 2-octenyl-1-thiol for hexanethiol, the title compound is obtained.

Example 37

[3α(Z),4α]-7-[4-[2-(Hexyloxy)ethyl]tetrahydro-3-furanyl]-5-heptenoic acid

A. [3α(Z),4α]-7-[4-[2-(2-Oxo)ethyl]tetrahydro-3-furanyl]-5-heptenoic acid, methyl ester

Into a dry 100 ml round bottom 3-necked flask containing a stir bar is added dried 3.27 g (9.54 mmoles) methoxymethyltriphenylphosphonium chloride ($(C_6H_5)_3P^+—CH_2OCH_3Cl^-$) and 30 ml distilled toluene (stored over molecular sieves). The resulting suspension is stirred in an ice-bath, under argon, until cold and then a 1.4 M solution of 5.73 ml (8.01 mmol) of potassium t-amylate in toluene is added dropwise. A bright red solution formed which is stirred at 0°C for an additional 35 minutes. Thereafter, a solution of 900 mg (3.75 mmol) [3α(Z),4α]-7-(4-formyl)tetrahydro-3-furanyl]-5-heptenoic acid, methyl ester (prepared as described in Example 1 Part A (16)) in 10 ml toluene is added by means of a dropping funnel over a 35 minute period with the ice-bath still in place. The reaction is then quenched by addition of 2.3 g (39 mmol) acetic acid in 5 ml ether. The reaction mixture is immediately poured into 200 ml saturated $NH_4Cl$, and extracted with ether (4 × 200 ml). The combined ether phases are washed with NaCl saturated solution, and dried ($MgSO_4$) and concentrated to yield an oil in a white crystalline solid (phosphine oxide). The white solid is triturated with EtOAc and the mother liquor is purified by chromatography on an LPS—1 silica column to obtain the enol-ether. The enol-ether is dissolved in 20 ml of THF and is then treated with 10 ml of a 20% aqueous trifluoroacetic acid solution. After 1 hour, trifluoroacetic acid is quenched by addition of solid $NaHCO_3$. The reaction mixture is extracted several times with methylene chloride. The methylene chloride extract is dried over anhydrous magnesium sulfate and concentrated under reduced pressure. The crude residue is chromatographed on a silica gel column to obtain the desired title A aldehyde.

B. [3α(Z),4α]-7-[4-[2-(Hydroxyethyl)]tetrahydro-3-furanyl]-5-heptanoic acid, methyl ester

The aldehyde (762 mg, 3 mmol) from part A in methanol (50 ml) is treated with $NaBH_4$ (0.11 g, 3 mmol) in an argon atmosphere at 0°C. After stirring at 0°C for 1 hour, the reaction is quenched by addition of 2N HCl (to pH 2). The methanol is removed *in vacuo* and the reaction mixture is taken up in ether. The ether solution is washed with saturated $KHCO_3$, saturated NaCl and dried ($MgSO_4$). The ether is evaporated to yield the title B compound.

C. [3α(Z),4α]-7-[4-[2-(Hexyloxy)ethyl]tetrahydro-3-furanyl]-5-heptenoic acid

Following the procedure of Example 1, Part C and Example 2 except substituting the above part B alcohol for the Example 1 Part B alcohol used in Example 1 Part C, the title compound is obtained.

Example 38

[3α(Z),4α]-7-[4-[2-(Hexylthio)ethyl]tetrahydro-3-furanyl]-5-heptenoic acid

Following the procedure of Examples 4 and 5 except substituting (3α,(Z)4α)-7-[4-[2-(2-oxo)ethyl]tetrahydro-3-furanyl]-5-heptenoic acid, methyl ester (prepared as described in Example 37 Part A) for the Example 1 Part A (16) aldehyde used in Example 4, Part A, the title compound is obtained.

Example 39

[3α(Z),4α]-7-[4-[2-(Benzyloxy)ethyl]tetrahydro-3-furanyl]-5-heptenoic acid

Following the procedure of Example 37 except substituting benzyl mesylate for hexyl mesylate, the title compound is obtained.

Example 40

[3α(Z),4α]-7-[4-[2-(Phenyloxy)ethyl]tetrahydro-3-furanyl]-5-heptenoic acid

Following the procedure of Example 6 except substituting Example 37 Part B alcohol for Example 1 Part B alcohol, the title acid is obtained.

Example 41

[3α(Z),4α]-7-[4-[2-(1-Butenyloxy)ethyl]tetrahydro-3-furanyl]-5-heptenoic acid

Following the procedure of Example 37 except substituting 1-butenyl mesylate for hexyl mesylate, the title compound is obtained.

Example 42

[3α(Z),4α]-7-[4-[2-(Cyclohexyloxy)ethyl]tetrahydro-3-furanyl]-5-heptenoic acid

Following the procedure of Example 37 except substituting cyclohexyl mesylate for hexyl mesylate, the title compound is obtained.

Example 43

[3α(Z),4α]-7-[4-[2-(Propyloxy)ethyl]tetrahydro-3-furanyl]-5-heptenoic acid

Following the procedure of Example 37 except substituting n-propyl mesylate for hexyl mesylate, the title compound is obtained.

Example 44

[3α(Z),4α]-7-[4-[2-(Cyclopentylmethyloxy)ethyl]tetrahydro-3-furanyl]-5-heptenoic acid

Following the procedure of Example 37 except substituting cyclopentylmethyl mesylate for hexyl mesylate, the title compound is obtained.

Example 45

[3α(Z),4α]-7-[4-[2-(Pentylthio)ethyl]tetrahydro-3-furanyl]-5-heptenoic acid

Following the procedure of Example 38 except substituting 1-pentanethiol for 1-hexanethiol, the title compound is obtained.

Example 46

[3α(Z),4α]-7-[4-[2-(Benzylthio)ethyl]tetrahydro-3-furanyl]-5-heptenoic acid

Following the procedure of Example 38 except substituting benzylmercaptan for hexanethiol, the title compound is obtained.

Example 47

[3α(Z),4α]-7-[4-[2-(Phenylthio)ethyl]tetrahydro-3-furanyl]-5-heptenoic acid

Following the procedure of Example 38 except substituting phenylmercaptan for hexanethiol, the title compound is obtained.

Example 48

[3α(Z),4α]-7-[4-[2-(Cyclohexylthio)ethyl]tetrahydro-3-furanyl]-5-heptenoic acid

Following the procedure of Example 38 except substituting cyclohexylmercaptan for hexanethiol, the title compound is obtained.

Example 49

[3α(Z),4α]-7-[4-[2-(Cyclohexylmethylthio)ethyl]tetrahydro-3-furanyl]-5-heptenoic acid

Following the procedure of Example 38 except substituting cyclohexylmethylmercaptan for hexanethiol, the title compound is obtained.

Example 50

[3α(Z),4α]-7-[4-[2-(1-Propenylthio)ethyl]tetrahydro-3-furanyl]-5-heptenoic acid

Following the procedure of Example 38 except substituting 1-(1-propenyl)thiol for 1-hexanethiol, the title compound is obtained.

Example 51

(3α,4α)-7-[4-[2-(Hexyloxy)ethyl]tetrahydro-3-furanyl]heptanoic acid

Following the procedure of Example 37 except substituting the Example 21 Part B aldehyde for the Example 1 Part A (16) aldehyde used in Example 37 Part A, the title compound is obtained.

Example 52

(3α,4α)-7-[4-[2-(Cyclopentylethyloxy)ethyl]tetrahydro-3-furanyl]heptanoic acid

Following the procedure of Example 37 except substituting the Example 21 Part B aldehyde for the Example 1 Part A (16) aldehyde used in Example 37 Part A and substituting cyclopentylethyl mesylate for hexyl mesylate, the title compound is obtained.

Example 53

(3α,4α)-7-[4-[2-(Benzyloxy)ethyl]tetrahydro-3-furanyl]heptanoic acid

Following the procedure of Example 37 except substituting the Example 21 Part B aldehyde for the Example 1 Part A (16) aldehyde used in Example 37 Part A and substituting benzyl mesylate for hexyl mesylate, the title compound is obtained.

Example 54

(3α,4α)-7-[4-[2-(Phenyloxy)ethyl]tetrahydro-3-furanyl]heptanoic acid

Following the procedure of Examples 37 and 6 except substituting the Example 21 Part B aldehyde for the Example 1 Part A (16) aldehyde used in Example 37 Part A and substituting the resulting (3α,4α)-7-[4-[2-(hydroxyethyl)]tetrahydro-3-furanyl]heptanoic acid, methyl ester for Example 1 Part B alcohol in Example 6 Part (a), the title acid is obtained.

Example 55

(3α,4α)-7-[4-[2-(Cyclopentyloxy)ethyl]tetrahydro-3-furanyl]heptanoic acid

Following the procedure of Example 37 except substituting the Example 21 Part B aldehyde for the Example 1 Part A (16) aldehyde used in Example 37 Part A and substituting cyclopentyl mesylate for hexyl mesylate, the title compound is obtained.

Example 56

(3α,4α)-7-[4-[2-(3-Hexenyloxy)ethyl]tetrahydro-3-furanyl]heptanoic acid

Following the procedure of Example 37 except substituting the Example 21 Part B aldehyde for the Example 1 Part A (16) aldehyde used in Example 37 Part A and substituting 3-hexenyl mesylate for hexyl mesylate, the title compound is obtained.

Example 57

(3α,4α)-7-[4-[2-(Cyclopropylmethylthio)ethyl]tetrahydro-3-furanyl]heptanoic acid

Following the procedure of Example 38 except substituting the Example 21 Part B aldehyde for the Example 37 Part A aldehyde and substituting cyclopropylmethyl mercaptan for 1-hexanethiol, the title compound is obtained.

Example 58

(3α,4α)-7-[4-[2-(Benzylthio)ethyl]tetrahydro-3-furanyl]heptanoic acid

Following the procedure of Example 38 except substituting the Example 21 Part B aldehyde for the Example 37 Part A aldehyde and substituting benzylmercaptan for 1-hexanethiol, the title compound is obtained.

Example 59

(3α,4α)-7-[4-[2-(Phenylthio)ethyl]tetrahydro-3-furanyl]heptanoic acid

Following the procedure of Example 38 except substituting the Example 21 Part B aldehyde for the Example 37 Part A (16) aldehyde and substituting phenylmercaptan for 1-hexanethiol, the title compound is obtained.

Example 60

(3α,4α)-7-[4-[2-(Cyclohexylthio)ethyl]tetrahydro-3-furanyl]heptanoic acid

Following the procedure of Example 38 except substituting the Example 21 Part B aldehyde for the Example 37 Part A aldehyde and substituting cyclohexylmercaptan for 1-hexanethiol, the title compound is obtained.

Example 61

(3α,4α)-7-[4-[2-(Heptenylthio)ethyl]tetrahydro-3-furanyl]heptanoic acid

Following the procedure of Example 38 except substituting the Example 21 Part B aldehyde for the Example 37 Part A (16) aldehyde and substituting 1-(2-heptenyl)thiol for 1-hexanethiol, the title compound is obtained.

Example 62

[3α(Z),4α]-7-[4-[4-(Hexyloxy)butyl]tetrahydro-3-furanyl]-5-heptenoic acid

A. [3α(Z),4α]-7-[4-[3-(3-Oxo)propyl]tetrahydro-3-furanyl]-5-heptenoic acid, methyl ester

Following the procedure of Example 37 Part A except substituting [3α(Z),4α]-7-[4-[2-(2-oxo)ethyl]-tetrahydro-3-furanyl]-5-heptenoic acid, methyl ester for [3α(Z),4α]-7-[(4-formyl)tetrahydro-3-furanyl]-5-heptenoic acid, methyl ester, the title A compound is obtained.

B. [3α(Z),4α]-7-[4-(4-Oxo)butyl]tetrahydro-3-furanyl]-5-heptenoic acid, methyl ester

Following the procedure of Example 37 Part A except substituting the aldehyde from Part A above for [3α(Z),4α]-7-[4-[2-(2-oxo)ethyl]tetrahydro-3-furanyl]-5-heptenoic acid, methyl ester, the title B aldehyde is obtained.

C. [3α(Z),4α]-7-[4-(4-Hydroxybutyl)]tetrahydro-3-furanyl]-5-heptenoic acid, methyl ester

Following the procedure of Example 37 Part B except substituting the title B aldehyde for [3α(Z),4α]-7-[4-[2-(2-oxo)ethyl]tetrahydro-3-furanyl]-5-heptenoic acid, methyl ester, the title C alcohol is obtained.

D. [3α(Z),4α]-7-[4-[4-(Hexyloxy)butyl]tetrahydro-3-furanyl]-5-heptenoic acid

Following the procedure of Example 1 except substituting the above part C alcohol for the alcohol used in Example 1 Part C, the title compound is obtained.

Example 63

[3α(Z),4α]-7-[4-[4-(Benzyloxy)butyl]tetrahydro-3-furanyl]-5-heptenoic acid

Following the procedure of Examples 62 and 37 except substituting the Example 62 Part C alcohol for the Example 37 Part A alcohol and substituting benzyl mesylate for hexyl mesylate, the title compound is obtained.

Example 64

[3α(Z),4α]-7-[4-[4-(Cyclohexylthio)butyl]tetrahydro-3-furanyl]-5-heptenoic acid

Following the procedure of Examples 62, 37 and 38 except substituting the Example 62 Part C alcohol for the Example 37 Part B alcohol and substituting cyclohexylmercaptan for 1-hexanethiol, the title compound is obtained.

Example 65

[3α(Z),4α]-7-[4-[(Hexylsulfinyl)methyl]tetrahydro-3-furanyl]-5-heptenoic acid and
[3α(Z),4α]-7-[4-[(Hexylsulfonyl)methyl]tetrahydro-3-furanyl]-5-heptenoic acid

To a solution of 562 mg (1.72 mmol) of [3α(Z),4α]-7-[4-[(hexylthio)methyl]tetrahydro-3-furanyl]-5-heptenoic acid, methyl ester (prepared as described in Example 4) in 6.78 ml of methanol at 0°C is added dropwise over 4 minutes 8.37 ml of 0.5M aqueous sodium periodate solution. Tetrahydrofuran (2 ml) is then added and the resulting reaction mixture is stirred at room temperature for 15 hours. A white precipitate is removed by filtration and washed with ether (3 × 50 ml). The filtrate is washed with 60 ml of saturated aqueous $NaHCO_3$ solution and dried over anhydrous magnesium sulfate. Concentration *in vacuo* affords 539 mg of an oily crude product. This is chromatographed on 54.16 g of silica gel 60 using 0.5—1.0% $CH_3OH$ to give the title compounds.

Example 66

[3α(Z),4α]-7-[4-[(Hexylsulfonyl)methyl]tetrahydro-3-furanyl]-5-heptenoic acid

To a stirred solution of 143 mg (0.4 mmol) of the Example 65 sulfonyl compound in 20.3 ml of THF and 3.09 ml of $H_2O$ under argon is added 3.90 ml of 1N aqueous lithium hydroxide solution. This mixture is purged with argon vigorously for 10 minutes and stirred at room temperature for 6 hours. The reaction mixture is acidified to pH 4 by addition of 1N aqueous HCl solution and poured into 30 ml of saturated NaCl solution. The resulting solution is saturated with solid NaCl and extracted with EtOAc (4 × 50 ml). The combined EtOAc extracts are dried ($MgSO_4$), filtered and concentrated *in vacuo* to give 140 mg of crude acid which is purified by flash chromatography.

Example 67

[3α(Z),4α]-7-[4-[(Hexylsulfinyl)methyl]tetrahydro-3-furanyl]-5-heptenoic acid

To a stirred solution of 120 mg (0.35 mmol) of Example 65 ester and sulfinyl ester in 27.0 ml of the THF and 4.11 ml of $H_2O$ under argon is added 5.19 ml of 1N aqueous lithium hydroxide solution. This mixture is purged with argon vigorously for 10 minutes and stirred at room temperature for 6 hours. The reaction

mixture is acidified to pH 4 by addition of 1N aqueous HCl solution and poured into 50 ml of saturated NaCl solution. The resulting solution is saturated with solid NaCl and extracted with EtOAc (4 × 100 ml). The combined EtOAc extracts are dried ($MgSO_4$), filtered and concentrated *in vacuo* to give crude acid which is purified by flash chromatography.

Example 68

[3α(Z),4β]-7-[4-[2-(Hexyloxymethyl)]tetrahydro-3-furanyl]-2,5-heptadienoic acid

A. [3α(Z),4β]-7-[4-[2-(Hexyloxymethyl)]tetrahydro-3-furanyl]-2-selenophenyl-5-heptenoic acid, methyl ester

To a solution of 308 μl of diisopropylamine (2.2 mmole) in 5 ml of dry THF, cooled at −78°C, is added dropwise 1.25 ml of a 1.6*M* solution of n-butyllithium in hexane. After 30 minutes at −78°C, a solution of 356 mg of [3α(Z),4β]-7-[4-[(hexyloxy)methyl]tetrahydro-3-furanyl]-5-heptenoic acid, hexyl ester, prepared as described in Example 2, (1 mmole) in 2 ml of dry THF is added dropwise. The reaction mixture is stirred for 30 minutes, whereupon a solution of 625 mg of diphenyldiselenide (2 mmole) in 2 ml of dry THF is added. The yellow color of diselenide disappears immediately upon its addition, initially. The yellow solution is stirred at −78°C for 30 minutes, whereupon the cooling bath was removed. The reaction mixture is then quenched by addition of aqueous ammonium chloride solution. It is then diluted with water and the organic layer is separated. The aqueous layer is extracted with ether. The combined organic extract is dried over anhydrous magnesium sulfate and concentrated *in vacuo*. Purification by chromatography on a silica gel column and eluting with 5—25% ethyl acetate in hexane gives 600 mg of title α-selenophenyl ester (90% yield).

B. [3α(Z),4β]-7-[4-[2-(Hexyloxymethyl)]tetrahydro-3-furanyl]-2-selenophenyl-5-heptenoic acid

A solution of 600 mg of title A α-selenophenyl esters in 10 ml of distilled THF is treated with 5 ml of a 1*N* aqueous lithium hydroxide solution. After stirring at room temperature for 2 days, the reaction mixture is acidified with 1*N* aqueous hydrochloric acid solution and extracted with methylene chloride. The methylene chloride extract is dried over anhydrous magnesium sulfate and concentrated *in vacuo* to yield 560 mg of title acid.

C. [3α(Z),4β]-7-[4-[2-(Hexyloxymethyl)]tetrahydro-3-furanyl]-2,5-heptadienoic acid

A solution of 560 mg of title B acid (0.86 mmole) in 10 ml of distilled THF is treated with 500 ml of a 30% aqueous hydrogen peroxide solution at 0—5°C. After a few minutes, the cooling bath is removed and the reaction mixture is stirred at room temperature for 1 hour. It is then diluted with methylene chloride and washed thoroughly with water. The organic layer is dried over anhydrous $MgSO_4$ and concentrated under reduced pressure. The crude residue is chromatographed on a CC-7 silica gel column and eluted with 20—60% ethyl acetate in hexane to obtain the title α,β-unsaturated acid.

Example 69

[3α(Z),4α]-7-[4-[2-(Hexylthio)methyl]tetrahydro-3-furanyl]-2,5-heptadienoic acid

Following the procedure of Example 68 except substituting the Example 4 compound for the Example 2 compound in Example 68 Part A, the title compound is obtained.

Example 70

[3α(Z),4α]-7-[4-[2-(Phenyloxy)methyl]tetrahydro-3-furanyl]-2,5-heptadienoic acid

Following the procedure of Example 68 except substituting the ester compound prepared in Example 6 for the Example 2 compound in Example 68, Part A, the title compound is obtained.

Example 71

[3α(Z),4α]-7-[4-[2-(Benzyloxy)methyl]tetrahydro-3-furanyl]-2,3-heptadienoic acid

Following the procedure of Example 68 except substituting the ester compound prepared in Example 7 for the Example 2 compound in Example 68 Part A, the title compound is obtained.

Example 72

[3α(Z),4α]-7-[4-[(2-Pentenyloxy)methyl]tetrahydro-3-furanyl]-2,5-heptadienoic acid

Following the procedure of Example 68 except substituting the ester compound prepared in Example 9 for the Example 2 compound in Example 68 Part A, the title compound is obtained.

Example 73

[3α(Z),4α]-7-[4-[(Heptyloxy)methyl]tetrahydro-3-furanyl]-2,5-heptadienoic acid

Following the procedure of Example 68 except substituting the ester compound prepared in Example 11 for the Example 2 compound in Example 68 Part A, the title compound is obtained.

Example 74

[3α(Z),4α]-7-[4-[(Cyclopentyloxy)methyl]tetrahydro-3-furanyl]-2,5-heptadienoic acid

Following the procedure of Example 68 except substituting the ester compound prepared in Example 13 for the Example 2 compound in Example 23 Part A, the title compound is obtained.

Example 75

[3α(Z),4β]-7-[4-[(Cyclohexyloxy)methyl]tetrahydro-3-furanyl]-2,5-heptadienoic acid

Following the procedure of Example 68 except substituting the ester compound prepared in Example 14 for the Example 2 compound in Example 68 Part A, the title compound is obtained.

Example 76

[3α(Z),4β]-7-[4-[(3-Butenyloxy)methyl]tetrahydro-3-furanyl]-2,5-heptadienoic acid

Following the procedure of Example 68 except substituting the ester compound prepared in Example 15 for the Example 2 compound in Example 68 Part A, the title compound is obtained.

Example 77

[3α(Z),4α]-7-[4-[(2-(Hexenylthio)methyl]tetrahydro-3-furanyl]-2,5-heptadienoic acid

Following the procedure of Example 68 except substituting the ester compound prepared in Example 16 for the Example 2 compound in Example 68 Part A, the title compound is obtained.

Example 78

[3α,4α]-7-[4-[(Hexyloxy)methyl)]tetrahydro-3-furanyl]-2,5-heptadienoic acid

Following the procedure of Example 68 except substituting the ester compound prepared in Example 21 for the Example 2 compound in Example 68 Part A, the title compound is obtained.

Example 79

(3α,4α)-7-[4-[(Octylthio)methyl]tetrahydro-3-furanyl]-2,5-heptenoic acid

Following the procedure of Example 68 except substituting the ester compound prepared in Example 31 for the Example 2 compound in Example 68 Part A, the title compound is obtained.

Example 80

[3α(Z),4α]-7-[4-[2-(Hexyloxy)ethyl]tetrahydro-3-furanyl]-2,5-heptadienoic acid

Following the procedure of Example 68 except substituting the ester compound prepared in Example 37 for the Example 2 compound in Example 68 Part A, the title compound is obtained.

Example 81

[3α(Z),4α]-7-[4-[2-(Hexylthio)ethyl]tetrahydro-3-furanyl]-2,5-heptadienoic acid

Following the procedure of Example 68 except substituting the ester compound prepared in Example 38 for the Example 2 compound in Example 68 Part A, the title compound is obtained.

Example 82

(3α,4α)-7-[4-[2-(Hexyloxy)ethyl]tetrahydro-3-furanyl]-2-heptenoic acid

Following the procedure of Example 68 except substituting the ester compound prepared in Example 51 for the Example 1 compound in Example 68 Part A, the title compound is obtained.

Example 83

(3α,4α)-7-[4-[2-(Cyclohexylthio)ethyl]tetrahydro-3-furanyl]-2-heptenoic acid

Following the procedure of Example 68 except substituting the ester compound prepared in Example 9 for the Example 1 compound in Example 68 Part A, the title compound is obtained.

Examples 84 to 93

Following the procedure as outlined in the specification and described in the working Examples, the following additional compounds may be prepared.

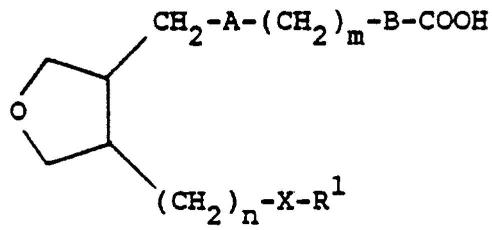

| Ex. No. | A | m | B | $(CH_2)_n$ | $R^1$ | X |
|---|---|---|---|---|---|---|
| 84. | CH=CH | 1 | CH=CH | $CH_2$ | $CH_3CH=CH$ | S |
| 85. | $(CH_2)_2$ | 2 | CH=CH | $(CH_2)_2$ | $CH_3CH_2CH=CHCH_2-$ | O |
| 86. | --- | 3 | CH=CH | $(CH_2)_3$ | 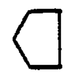 | S=O |
| 87. | CH=CH | 4 | --- | $-\underset{CH_3}{CH}CH_2-$ | 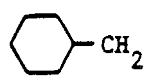 | O=S=O |
| 88. | $(CH_2)_2$ | 5 | CH=CH | $(CH_2)_4$ | $C_6H_5$ | S |
| 89. | CH=CH | 6 | -- | $(CH_2)_5$ | $C_6H_5(CH_2)_2$ | O |
| 90. | $\underset{CH_3}{CH}-CH_2$ | 7 | --- | $(CH_2)_6$ | $C_6H_5CH_2$ | S |
| 91. | $CH_2CH$ | 8 | CH=CH | $(CH_2)_7$ | $C_6H_{13}$ | S=O |
| 92. | $(CH_2)_2$ | 6 | CH=CH | $(CH_2)_8$ | $C_7H_{15}$ | O=S=O |
| 93. | --- | 2 | --- | $(CH_2)_2$ | $C_2H_5$ | O |

The following Examples represent preferred embodiments of the invention. Unless otherwise indicated, all temperatures are expressed in degrees Centigrade.

Example 94

[3α(Z),4α]-7-[4-[(Hexyloxy)methyl]tetrahydro-3-thienyl]-5-heptenoic acid

A. [3α(Z),4α]-7-[Tetrahydro-4-formyl-3-thienyl]-5-heptenoic acid, methyl ester

(1) (1α,2β,3β)-1-Trimethylsilyloxy-cyclohex-5-ene, 2,3-dicarboxylic acid anhydride

To a solution of 23.6 ml of 1-trimethylsilyloxybutadiene (133.33 mmol) in 200ml of dry methylene chloride was added with stirring 10 g of maleic anhydride (100 mmole). The homogeneous reaction mixture was stirred at room temperature for 24 hours, whereupon most of the methylene chloride was removed by distillation under reduced pressure. The crude oil was presoaked in silica gel and loaded on a 200 g silica gel column. Elution with 10—25% ethyl acetate in hexane and finally with 50% ethyl acetate in hexane gave 23.13 g of desired title adduct as a colorless oil.

(2) (1α,2β,3β)-1-Hydroxy-cyclohex-5-ene-2,3-dicarboxylic acid anhydride

To a solution of 10 g of Part (1) tri-methylsilyloxy adduct (41.7 mmole) in 50 ml of distilled THF was added with stirring 1 ml of concentrated hydrochloric acid. The reaction mixture was stirred at room temperature for 2 hours, whereupon it was filtered through a pad of anhydrous magnesium sulfate. The filtrate was concentrated under reduced pressure obtain the title desilylated adduct.

(3) (1α,2β,3β)-1-Tetrahydropyranyloxycyclohex-5-ene-2,3-dicarboxylic acid anhydride

The crude Part (2) desilylated adduct was dissolved in 50 ml of dry methylene chloride and cooled in an ice-water bath. To this solution was added 5.6 ml of reagent grade dihydropyran, followed by 30 mg of p-toluene sulfonic acid. After stirring for 1 hour at 0—5°C, the reaction mixture was washed with saturated sodium bicarbonate solution. The aqueous layer was extracted with ether. The combined organic extract was dried over anhydrous magnesium sulfate and concentrated under reduced pressure. The crude residue was chromatographed on a silica gel column and eluted with 10—20% ethyl acetate in hexane to obtain 9.33 g of desired title tetrahydropyranyl ether as a colorless oil (93% yield).

(4) (1α,2β,3β)-1-Tetrahydropyranyloxycyclohex-5-ene-2,3-dimethanol

To a suspension of 2.28 g of 95% pure lithium aluminum hydride in 300 ml of freshly distilled THF (60 mmole), cooled in an ice-water bath was added dropwise a solution of 9.33 g of Part (3) anhydride (38.2 mmole) in 50 ml of dry THF over a period of 45 minutes. After the addition was complete, the cooling bath was removed and the reaction mixture was allowed to stand at room temperature overnight, whereupon it was again placed on an ice-water bath and excess of LAH was destroyed by careful addition of freshly prepared saturated sodium sulfate solution. Addition of sodium sulfate was continued until all the lithium and aluminum salts were precipitated as a granular solid. Solid magnesium sulfate was added to the reaction mixture and it was then filtered. The residue was washed several times with methylene chloride. Finally the residue was taken up in 1000 ml of a 10% acetonitrile in ethyl acetate and stirred for 30 minutes. It was then filtered. Combined filtrate was concentrated under reduced pressure to obtain any oily residue. The crude residue was chromatographed on a silica gel column and eluted with 20—50% ethyl acetate in hexane to obtain 8.17 g of desired title diol as a viscous oil (~93% yield).

(5) (1α,2β,3β)-1-Tetrahydropyranyloxycyclohex-5-ene-2,3-dimethanolbismethyl carbonate

To a solution of 7.767 g of Part (4) diol (31.83 mmole) in 100 ml of dry methylene chloride and 10 ml of pyridine (250 mmole) cooled at 0°C was added with stirring 390 mg of 4-dimethylamino pyridine (3.2 mmole, 10 mole%) followed by 5.8 ml of methyl chloroformate (75 mmole, 1.17 equiv) dropwise. An immediate precipitate of pyridinium hydrochloride was observed. The reaction mixture was maintained at 0°C for additional 4 hours, whereupon it was washed thoroughly with water and then with saturated copper sulfate solution. The aqueous extracts were extracted with ether (X3).

The combined organic extract was washed with water, saturated salt solution and finally was dried over magnesium sulfate. Evaporation of solvent under reduced pressure gave an oil which was chromatographed on a 250 g silica gel column and eluted with 5—15% ethyl acetate in hexane to obtain 10.87 g of desired title bis-methylcarbonate (95% yield) which solidified on standing in the cold room.

(6) (1α,2β,3β)-1-Tetrahydropyranyloxy-5,6-dihydroxycyclohexane-2,3-dimethanolbismethyl carbonate

To a solution of 5.13 g of Part (6) bis-methylcarbonate (14.25 mmole) in 30 ml of distilled THF was added with stirring 2.16 g of crystalline N-methylmorpholine N-oxide (16 mmole). Water was added dropwise to the reaction mixture, until it became homogeneous. To this homogeneous solution was now added 100 ml of a solution of osmium tetroxide (250 mg/5 ml of ether) in ether. The reaction mixture was stirred at room temperature for 30 hours, whereupon aqueous sodium bisulfate solution was added to the reaction mixture. The solution was stirred for an additional 30 minutes, whereupon the organic layer was separated and the aqueous layer was extracted several times with methylene chloride. The combined organic extract was washed with saturated salt solution, dried over anhydrous magnesium sulfate and finally was concentrated under reduced pressure. Trituration with ether gave a white precipitate which was filtered off and washed with cold-ether. 3.88 g of crystalline diol was obtained as white solid. The filtrate was concentrated under reduced pressure and the residue was chromatographed on a silica gel column. Elution with 30—50% ethyl acetate in hexane and finally with ethyl acetate gave an additional 987 mg of crystalline title diol. Total yield = 4.867 g (~87% yield).

(7) [2-(1-Formyl-methyl)-3-(1-tetrahydropyranyloxy-1-formyl-methyl)butane]-1,4-bismethyl carbonate

To a solution of 3.67 g of crystalline Part (6) diol (~10 mmole) in 15 ml of methanol and 15 ml of distilled THF, cooled in an ice-water bath was added with stirring a solution of 1.75 g of powdered sodium-metaperiodate (15 mmole) in 15 ml of water. After the addition was over, the reaction mixture was stirred vigorously at 0—5°C for 1 hour and finally at room temperature for an additional 4 hours, whereupon TLC indicated complete disappearance of the diol. The crude reaction mixture was diluted with ether and washed thoroughly with water. The aqueous layer was reextracted with ether (X3). The combined organic extract was dried over anhydrous magnesium sulfate and finally was concentrated under reduced pressure to obtain the crude title dialdehyde as a colorless oil.

(8) [2-(1-Hydroxymethyl-methyl)-3-(1-tetrahydropyranyloxy-1-hydroxymethylmethyl)butane]-1,4-bismethyl carbonate

The crude Part (7) dialdehyde was now dissolved in 25 ml of methanol and cooled at −10°C in a dry ice-acetone bath and 380 mg of solid sodium borohydride (10 mmole) was added in portions with stirring. After stirring at −10°C to +5°C for 1 hour, aqueous ammonium chloride solution was added to the reaction mixture. It was then extracted with ether (X3) and then with methylene chloride (X3). The combined organic extract was dried over anhydrous magnesium sulfate and finally was concentrated under reduced pressure to obtain 3.83 g of crude title diol as a viscous oil.

(9) [2-(2-Hydroxyethyl)3-(3,3-dimethyl-2,4-dioxa-cyclopentyl)-butane]-1,4-bismethyl carbonate

To a solution of 3.83 g of crude Part (8) diol from the previous reaction in 20 ml of dry methanol and 20 ml of dry acetone (dried over molecular sieves) was added with stirring 800 mg of powdered and dry Amberlyst-15 acid resin. The heterogeneous reaction mixture was stirred under an argon atmosphere overnight, whereupon it was diluted with ether and filtered through anhydrous magnesium sulfate. Residual molecular Amberlyst resin was thoroughly washed with ether. The filtrate was concentrated under reduced pressure and the crude residue was chromatographed on a silica gel column. Elution with 20—50% ethyl acetate in hexane gave 2.85 g of desired title acetonide alcohol (87% overall yield from six membered Part (6) diol) as a colorless viscous oil.

(10) [2-(2-Tetrahydropyranyloxyethyl)-3-(3,3-dimethyl-2,4-dioxa-cyclopentyl)butane]-1,4-bismethyl carbonate

To a solution of 2.65 g of Part (9) acetonide-alcohol (7.6 mmole) in 40 ml of dry methylene chloride was added with stirring at 0—5°C (ice-water bath) a catalytic amount of p-toluene sulfonic acid and 750 µl of dihydropyran (8.3 mmole). The reaction mixture was stirred under dark at 0—5°C for 1 hour, whereupon it was washed with aqueous sodium bicarbonate solution. The aqueous layer was extracted with ether (X2). The combined organic extract was dried over anhydrous magnesium sulfate, filtered and the filtrate was concentrated under reduced pressure. The crude oily residue was chromatographed on a silica-gel column and eluted with 5—10% ethyl acetate in hexane to obtain 3.02 g of desired title tetrahydropyranyl ether which crystallized on standing at −20°C (92% yield).

(11) [2-(2-Tetrahydropyranyloxyethyl)-3-(3,3-dimethyl-2,4-dioxa-cyclopentyl)butane]-1,4-diol

To a suspension of 380 mg of lithium aluminum hydride (10 mmole) in 15 ml of freshly distilled THF, cooled in an ice-water bath was added with stirring, dropwise a solution of 2.75 g of Part (9) bis-carbonate (6.3 mmole) in 10 ml of dry THF over a period of 15 minutes. After the addition was over, the reaction mixture was stirred at 0—5°C and finally at room temperature for 3 hours, whereupon it was placed in a cold-bath and an excess of hydride was carefully decomposed by slow addition of saturated sodium sulfate solution. Addition of saturated sodium sulfate solution was continued until all the inorganic salts were precipitated as a white granular solid. Solid magnesium sulfate was added to the reaction mixture and it was then filtered. The residue was thoroughly washed with THF and methylene chloride (1:1). The combined filtrate was concentrated under reduced pressure. The crude oily residue was chromatographed on a silica gel column and eluted with 50% ethyl acetate in hexane followed by ethyl acetate to obtain 1.85 g of desired title diol as a viscous oily residue (92.5% yield).

(12) [2-(2-Tetrahydropyranyloxyethyl)-3-(3,3-dimethyl-2,4-dioxa-cyclopentyl)butane]-1,4-bismethanesulfonate

To a solution of 960 µl of methanesulfonylchloride (12 mmole) in 10 ml of pyridine, cooled at −10°C in a dry ice-acetone bath was added with stirring, a solution of 1.61 g of Part (II) diol (5.07 mmole) in 2 ml of pyridine and 5 ml of methylene chloride, dropwise over a period of 10 minutes. After the addition was over, the reaction mixture was allowed to warm to 0°C and left at 0—5°C for 3 hours, whereupon it was diluted with ether and washed thoroughly with water and saturated copper sulfate solution to remove pyridine. The aqueous wash was extracted with ether (X3). The combined organic extract was washed with water, dried over anhydrous magnesium sulfate and concentrated under reduced pressure. The crude oily title bis-mesylate residue was further dried *in vacuo* and was then immediately used in the next reaction.

(13) (3α,4α)-2-[Tetrahydro-4-(4,4-dimethyl-3,5-dioxa-cyclopentyl)-3-thienyl]ethanol, tetrahydropyranyloxy ether

2.4 g of recrystallized sodium sulfate nonahydrate (crystallized from hot ethanol) was added to 70 ml of dry dimethyl sulfoxide. Roughly 25 ml of dimethyl sulfoxide was removed by distillation under reduced pressure (bath temperature 90°C). The reaction mixture was cooled to room temperature and the distillation head was replaced with a reflux condenser. A solution of the crude Part (12) bis-mesylate (~5.07 mmole) in 5 ml of dimethyl sulfoxide and 5 ml of ether was added dropwise with stirring over a period of 5 minutes. The reaction mixture was now heated to 70°C and maintained at that temperature for 3 hours, whereupon it was cooled, diluted with ether and washed thoroughly with water. The aqueous layer was reextracted with ether (X2). The combined ether extract was dried over anhydrous magnesium sulfate and finally was concentrated under reduced pressure. The crude residue was chromatographed on a silica gel column and eluted with 5—15% ethyl acetate in hexane to obtain 1.36 g of desired title tetrahydrothiophane as an oil. 86% yield in two steps from diol).

(14) (3α,4α)-2-[Tetrahydro-4-(4,4-dimethyl-3,5-dioxa-cyclopentyl)-3-thienyl]ethanol

To a solution of 1.56 g of Part (13) tetrahydrothiophene-THP ether (4.93 mmole) in 10 ml of dry methanol and 10 ml of dry acetone was added with stirring 450 mg of dried and crushed Amberlyst-15 resin. The reaction mixture was stirred at room temperature under an argon atmosphere for 6 hours, whereupon it was diluted with ether and filtered through anhydrous magnesium sulfate. The filtrate was concentrated under reduced pressure and the crude oily residue was chromatographed on a silica gel

column. Elution with 30% ethyl acetate in hexane, followed by 50% ethyl acetate in hexane and finally with ethyl acetate gave 1.06 g of desired title alcohol as a white crystalline solid (92.5% yield).

(15) (3α,4α)-2-[Tetrahydro-4-(4,4-dimethyl-3,5-dioxa-cyclopentyl)-3-thienyl]acetaldehyde

To a solution of 400 μl of distilled oxalylchloride (5 mmole) in 10 ml of anhydrous methylene chloride, cooled at −78°C in a dry ice-acetone bath was added with stirring, dropwise 800 μl of dry dimethylsulfoxide (11.2 mmole) over a period of 10 minutes. A rapid gas evolution occurred during its addition. After 20 minutes at −78°C, a solution of 712 mg of Part (14) alcohol (2.93 mmole) in 5 ml of methylene chloride was added dropwise at −78°C over a period of 5 minutes. Additional stirring was continued for 30 minutes, whereupon 1.5ml of distilled triethylamine was added at −78°C. After stirring at −78°C for 20 minutes, the cooling bath was removed and the reaction mixture was warmed to 0°C, whereupon water was added to the reaction mixture. The reaction mixture was allowed to stand at room temperature for an additional 5 minutes, whereupon it was diluted with ether and washed thoroughly with water. The aqueous layer was extracted with ether (X3). The combined ether extract was dried over anhydrous magnesium sulfate and finally was concentrated under reduced pressure to obtain 727 mg of title aldehyde as an oily residue. This was used in the Wittig reaction without any additional purifications.

(16) [3α(Z),4α]-7-[Tetrahydro-4-(4,4-dimethyl-3,5-dioxa-cyclopentyl)-3-thienyl]-5-heptenoic acid and
(17) [3α(Z),4α]-7-[Tetrahydro-4-(4,4-dimethyl-3,5-dioxa-cyclopentyl)-3-thienyl]-5-heptenoic acid, methyl ester

To a suspension of 2.66 g of carboxybutyltriphenylphosphonium bromide (6 mmole) in 20 ml of freshly distilled THF, cooled in an ice-water bath was added with stirring 7.2 ml of 1.4 M solution of K-t-amylate in toluene, dropwise. After the addition, the water bath was removed and the orange yield suspension was stirred at room temperature for an additional 2 hours. It was again cooled in an ice-water bath and a solution of 727 mg of crude title (15) aldehyde (2.93 mmole) in 5 ml of dry THF was added dropwise. The reaction mixture was stirred at room temperature for an additional 1 hour, whereupon it was quenched by addition of glacial acetic acid. The reaction mixture was now diluted with ether and washed successively with water and saturated sodium bicarbonate solution (X3). The combined aqueous layer was extracted with ether (X2). The combined ether extract was dried over anhydrous magnesium sulfate and finally was concentrated under reduced pressure. The crude residue was triturated with ether and the precipitated phosphine oxide was filtered off. The filtrate was placed in a cold-water bath and etheral diazomethane solution was added with stirring until the yellow diazomethane color persisted for 15 minutes. Excess diazomethane was now removed by bubbling argon through the reaction mixture. It was now concentrated under reduced pressure and the crude residue was chromatographed on a silica gel column. Elution with 10—30% ethyl acetate in hexane gave 683 mg of desired title Wittig addition product (70% yield from alcohol) contaminated with ~15% of the undesired *E*-olefin.

(18) [3α(Z),4α]-7-[Tetrahydro-4-(1,2-dihydroxyethyl)-3-thienyl]-5-heptenoic acid, methyl ester

To a solution of 683 mg of Part (17) acetonide (1.93 mmole) in 10 ml of anhydrous methanol was added with stirring a catalytic amount of p-toluene sulfonic acid (~5 mg). The reaction mixture was stirred at room temperature under an argon atmosphere for 24 hours, whereupon it was concentrated under reduced pressure and the crude residue was chromatographed on a silica gel column. Elution with 20% ethyl acetate in hexane gave 110 mg of unreacted acetonide. Further elution with 50% ethyl acetate in hexane and finally with ethyl acetate gave 427 mg of desired title diol (84% yield based on a recovered acetonide) as a colorless oil.

(19) [3α(Z),4α]-7-[Tetrahydro-4-formyl-3-thienyl]-5-heptenoic acid, methyl ester

To 90 mg of Part (18) diol (0.31 mmole) in 2 ml of methanol at 25°C was added a solution of 75 mg of sodium metaperiodate (0.34 mmole, 1.1 equiv.) in 1 ml of $H_2O$. After stirring at 25°C for 1 hour, the reaction mixture was concentrated. The residue was diluted with 3 ml of $H_2O$, then extracted with three 10 ml portions of $CH_2Cl_2$. The combined organic layer was dried over anhydrous $MgSO_4$ and concentrated to give 80 mg of crude title aldehyde as a yellow oil. This was used immediately in the next reaction.

B. [3α(Z),4α]-7-[4-Hydroxymethyltetrahydro-3-thienyl]-5-heptenoic acid, methyl ester.

To a solution of Part A aldehyde (ca. 1.05 mmole) in 3 ml of methanol at 0°C was added 40 mg of sodium borohydride (1.05 mmole, 4 equiv.). After stirring at 0°C for 15 minutes, the reaction mixture was poured into 15 ml of a saturated $NH_4Cl$ solution. The aqueous solution was extracted with four 10 ml portions of ether. The combined ethereal extract was dried over anhydrous $MgSO_4$ and concentrated. The residue was purified on a silica gel column, with 35% ethyl acetate/hexanes as eluting solvents, to give 97 mg of title alcohol as an oil.

C. [3α(Z),4α]-7-[4-[(Hexyloxy)methyl]tetrahydro-3-thienyl]-5-heptenoic acid, hexyl ester

A mixture of 50 mg of powdered potassium hydroxide (0.9 mmole, 10 equiv.) in 10 ml of dry xylene was distilled off until ca. 5 ml of solution remained. To the cooled remaining solution was added a solution

of 20 mg of Part B alcohol (0.09 mmole) in 5 ml of xylene, and the resulting mixture was again distilled off to a volume of ca. 5 ml, then cooled to 25°C. A solution of 155 mg of hexylmesylate in 5 ml of dry xylene (0.9 mmole, 10 equiv.) was added and the reaction mixture was heated at reflux for 1 hour. The mixture was then cooled to 25°C and diluted with 30 ml of ether. The ethereal solution was washed with three 10 ml portions of $H_2O$, dried over anhydrous $MgSO_4$, and concentrated. The residue was purified on a silica gel column, with 5% ethyl acetate in hexanes as eluting solvents, to give 40 mg of the desired title ether, contaminated with some unidentified product.

Example 95

[3α(Z),4α]-7-[4-[(Hexyloxy)methyl]tetrahydro-3-thienyl]-5-heptenoic acid

To a solution of 84 mg of impure Example 94 ester (ca. 0.2 mmole) in 8 ml of dry THF, saturated with argon at 25°C was added 2 ml of 1*N* lithium hydroxide solution (2 mmole, 10 equiv.). After stirring at 25°C for 3 days, the reaction mixture was concentrated. The residue was diluted with 5 ml of $H_2O$, acidified to pH 3 with a saturated solution of oxalic acid, and extracted with three 10 ml portions of ether. The combined ethereal extract was washed with two 10 ml portions of $H_2O$, dried over anhydrous $MgSO_4$, and concentrated. The residue was purified on a CC—7 silica gel column, with a gradient of ether/pentanes as eluting solvents, to give 37.3 mg of title acid as a light yellow oil.

TLC: Silica gel; 5% $MeOH/CH_2Cl_2$; $R_f$ ~0.6

| | | | |
|---|---|---|---|
| Anal Calcd for $C_{18}H_{32}O_3S$: | C, 65.81; | H, 9.81; | S, 9.76 |
| Found: | C, 65.67; | H, 9.68; | S, 9.48 |

Example 96

[3α(Z),4α]-7-[4-[(Hexylthio)methyl]tetrahydro-3-thienyl]-5-heptenoic acid, methyl ester

A. [3a(Z),4a]-7-[4-Tetrahydro-4-tosyloxy-3-thienyl]heptenoic acid, methyl ester

To a solution of 50 mg of [3α(Z),4α]-7-[4-hydroxymethyltetrahydro-3-thienyl]-5-heptenoic acid, methyl ester (prepared as described in Example 94 Part B) (0.19 mmole) in 1 ml of pyridine at 25°C was added 90 mg of p-toluenesulfonyl chloride (0.38 mmole, 2 equiv.). After stirring at 25°C for 2 hours, the reaction mixture was diluted with 20 ml of ether. The ethereal solution was washed with three 5 ml portions of a saturated cupric sulfate solution, two 5 ml portions of $H_2O$, dried over anhdrous $MgSO_4$ and concentrated. The residue was purified on a silica gel column, with 5% ethyl acetate/hexanes (200 ml) and 25% ethyl acetate/hexanes (200 ml) as eluting solvents, to give 60 mg of title tosylate as a clear oil.

B. [3α(Z),4α]-7-[4-[(Hexylthio)methyl]tetrahydro-3-thienyl]-5-heptenoic acid, methyl ester

To a solution of 18 mg of potassium t-butoxide (0.16 mmole, 1.1 equiv.) in 3 ml of dry THF at 25°C under an argon atmosphere was added 90 mg of 1-hexanethiol (0.8 mmole, 5 equiv.). After stirring at 25°C for 30 minutes, a solution of 60 mg of Part A tosylate (0.14 mmole) in 1 ml of THF was added and the reaction was heated at reflux for 1 hour. The cooled reaction mixture was diluted with 30 ml of ether. The ethereal solution was washed with three 5 ml portions of a saturated $NaHCO_3$ solution and 5 ml of $H_2O$, then dried over anhydrous $MgSO_4$ and concentrated. The residue was purified on a silica gel column with 5% EtOAc/hexanes (100 ml) and 10% EtOAc/hexanes (100 ml) as eluting solvents, to give 51 mg of title thioether as a light yellow oil (contaminated with some hexane disulfide).

Example 97

[3α(Z),4α]-7-[4-[(Hexylthio)methyl]tetrahydro-3-thienyl]-5-heptenoic acid

To a solution of 51 mg of crude Example 96 ester (ca. 0.14 mmole) in 4.2 ml of THF, saturated with argon, at 25°C was added 1.4 ml of a 1*N* lithium hydroxide solution. After stirring at 25°C for 20 hours, the reaction mixture was concentrated. The residue was diluted with 5 ml of $H_2O$, acidified to pH 3 with a saturated solution of oxalic acid, then extracted with three 10 ml portions of ether. The combined ethereal extract was washed with two 5 ml portions of $H_2O$, dried over anhydrous $MgSO_4$, and concentrated. The residue was purified on a CC-7 silica gel column, with a gradient of pentane/ether as eluting solvents, to give 36 mg of title compound as a light yellow oil.

TLC: Silica gel; 5% $MeOH/CH_2Cl_2$, $R_f$ ~0.55

| | | | |
|---|---|---|---|
| Anal Calcd for $C_{18}H_{32}O_2S_2$: | C, 62.74; | H, 9.36; | S, 18.61 |
| Found: | C, 62.41; | H, 9.36; | S, 18.43 |

Example 98

[3α(Z),4β]-7-[4-[(Hexyloxy)methyl]tetrahydro-3-thienyl]-5-heptenoic acid, hexyl ester

A. [3α(Z),4β]-7-[Tetrahydro-4-formyl-3-thienyl]-5-heptenoic acid, methyl ester

To 140 mg of [3α(Z),4α]-7-[tetrahydro-4-formyl-3-thienyl]-5-heptenoic acid, methyl ester (prepared as described in Example 94, Part A (19) (0.58 mmole) in 2 ml of methanol is added 3.15 mg of sodium methoxide (58 μmole, 10%). After stirring at 25°C for 2 hours, the reaction mixture is poured into 50 ml of a saturated aqueous ammonium chloride solution and extracted with three 10 ml portions of ether. The organic layer is washed with 10 ml of $H_2O$ and dried over anhydrous $MgSO_4$ and concentrated to give 130 mg of title aldehyde as an oil. This was used without purification.

B. [3α(Z),4β]7-[Tetrahydro-4-hydroxymethyl-3-thienyl]-5-heptenoic acid, methyl ester

To 43 mg of title A aldehyde (0.18 mmole) in 1 ml of methanol at 0°C is added 6.8 mg of sodium borohydride (0.18 mmole, 4 eq.). After stirring at 0°C for 10 minutes, the mixture is poured into 20 ml of a saturated $NH_4Cl$ solution and extracted with three 10 ml portions of ether. The combined ethereal extract is dried over anhydrous $MgSO_4$ and concentrated to give 44 mg of title alcohol as an oil.

C. [3α(Z),4β]-7-[4-[(Hexyloxy)methyl]tetrahydro-3-thienyl]-5-heptenoic acid, hexyl ester

To 100 mg of powdered potassium hydroxide (1.8 mmole, 10 eq.) in 20 ml of dry xylene is added a solution of 44 mg of title B alcohol (0.18 mmole) in 20 ml of dry xylene. The mixture is heated to reflux and *ca.* 20 ml of xylene is distilled off.

To the cooled remaining solution is added a solution of 309 mg of hexyl mesylate (1.8 mmole, 10 eq.) and the mixture is heated at reflux for 3 hours. The mixture is cooled to 25°C, diluted with 100 ml of ether and washed with two 20 ml portions of water. The organic layer is dried over anhydrous $MgSO_4$ and concentrated to give an oil which is purified on a silica gel column, eluting with 10% EtOAc/hexanes to yield 45 mg of title ester as a yellow oil.

Example 99

[3α(Z),4β]-7-[4-[(Hexyloxy)methyl]tetrahydro-3-thienyl]-5-heptenoic acid

To 45 mg of Example 98 title C ester (0.14 mmole) in 6 ml of THF is added 1.6 ml of 1*M* lithium hydroxide solution. The mixture is stirred at 25°C for 4 days and then concentrated. The residue is diluted with 10 ml of $H_2O$ and acidified with a saturated oxalic acid solution to pH 3 and extracted with three 10 ml portions of ether. The combined ethereal extract is washed with two 10 ml portions of water, dried over anhydrous $MgSO_4$ and concentrated.

The residue is purified on a CC-7 silica gel column, eluting with a gradient of pentane/ether. The product is kept under high vacuum for 2 days to yield 28 mg of title compound as an oil.

Example 100

[3α(Z),4α]-7-[4-[(Phenyloxy)methyl]tetrahydro-3-thienyl]-5-heptenoic acid

(a) Phenol (1 mmole) is added to a solution of 262 mg triphenylphosphine (1 mmole), diethylazodicarboxylate (1 mmole) and Example 94, Part B (1 mmole) alcohol in 25 ml of dry THF and is stirred at 23°C for 48 hours. The reaction mixture is concentrated *in vacuo* and the residue is triturated with ether. Filtration, concentration of the filtrate under reduced pressure and finally chromatography of the residue on a silica gel column gives [3α(Z),4α]-7-[4-[(phenyloxy)methyl]tetrahydro-3-thienyl]-5-heptenoic acid, methyl ester.

(b) Following the procedure set out in Example 99, the ester from part (a) is converted to the title acid.

Example 101

[3α(Z),4α]-7-[4-[(Benzyloxy)methyl]tetrahydro-3-thienyl]-5-heptenoic acid

Following the procedure of Example 94 except substituting benzyl mesylate for hexyl mesylate, the title compound is obtained.

Example 102

[3α(Z),4α]-7-[4-[(Cyclohexyloxy)methyl]tetrahydro-3-thienyl]-5-heptenoic acid

Following the procedure of Example 94 except substituting cyclohexyl mesylate for hexyl mesylate, the title compound is obtained.

Example 103

[3α(Z),4α]-7-[4-[(2-Pentenyloxy)methyl]tetrahydro-3-thienyl]-5-heptenoic acid

Following the procedure of Example 94 except substituting 2-pentenyl-1-mesylate for hexyl mesylate, the title compound is obtained.

Example 104

[3α(Z),α]-7-[4-[(Cyclopentylmethyloxy]methyl]tetrahydro-3-thienyl]-5-heptenoic acid

Following the procedure of Example 94 except substituting cyclopentyl mesylate for hexyl mesylate, the title compound is obtained.

Example 105

[3α(Z),4β]-7-[4-[(Heptyloxy)methyl]tetrahydro-3-thienyl]-5-heptenoic acid

Following the procedure of Examples 95 and 96 except substituting heptyl mesylate for hexyl mesylate, the title compound is obtained.

Example 106

[3α(Z),4β]-7-[4-[(Phenethyloxy)methyl]tetrahydro-3-thienyl]-5-heptenoic acid

Following the procedure of Examples 98 and 99 except substituting phenethyl mesylate for hexyl mesylate, the title compound is obtained.

Example 107

[3α(Z),4β]-7-[4-[(Cyclopentyloxy)methyl]tetrahydro-3-thienyl]-5-heptenoic acid

Following the procedure of Examples 98 and 99 except substituting cyclopentyl mesylate for hexyl mesylate, the title compound is obtained.

Example 108

[3α(Z),4β]-7-[4-[(Cyclohexyloxy)methyl]tetrahydro-3-thienyl]-5-heptenoic acid

Following the procedure of Examples 98 and 99 except substituting cyclohexyl mesylate for hexyl mesylate, the title compound is obtained.

Example 109

[3α(Z),4β]-7-[4-[(3-Butenyloxy)methyl]tetrahydro-3-thienyl]-5-heptenoic acid

Following the procedure of Examples 98 and 99 except substituting for hexyl mesylate, the title compound is obtained.

Example 110

[3α(Z),4α]-7-[4-[(2-Hexenylthio)methyl]tetrahydro-3-thienyl]-5-heptenoic acid

Following the procedure of Examples 96 and 97 except substituting 2-hexenyl-1-thiol for hexane thiol, the title compound is obtained.

Example 111

[3α(Z),4α]-7-[4-[(Propylthio)methyl]tetrahydro-3-thienyl]-5-heptenoic acid

Following the procedure of Examples 96 and 97 except substituting propylthio for hexanethiol, the title compound is obtained.

Example 112

[3α(Z),4α]-7-[4-[(Cycloheptylthio)methyl]tetrahydro-3-thienyl]-5-heptenoic acid

Following the procedure of Examples 96 and 97 except substituting cycloheptylmercaptan for hexanethiol, the title compound is obtained.

Example 113

[3α(Z),4α]-7-[4-[(Benzylthio)methyl]tetrahydro-3-thienyl]-5-heptenoic acid

Following the procedure of Examples 96 and 97 except substituting benzylmercaptan for hexanethiol, the title compound is obtained.

Example 114

[3α(Z),4α]-7-[4-[(Phenylthio)methyl]tetrahydro-3-thienyl]-5-heptenoic acid

Following the procedure of Examples 96 and 97 except substituting phenylmercaptan for hexanethiol, the title compound is obtained.

Example 115

(3α,4α)-7-[4-[(Hexyloxy)methyl]tetrahydro-3-thienyl]heptanoic acid

A. [3α,4α(1S)]-7-[Tetrahydro-4-[(1-hydroxymethyl)methyl]-3-furanyl]heptanoic acid, methyl ester

A mixture of 600 mg of Example 94 Part A (18; diol, 100 mg of a 10% palladium over carbon in 80 ml of EtOAc and 4 ml of glacial acetic acid is shaken in a Parr bottle under 50lb. of hydrogen pressure at 25°C for 24 hours. The mixture is then filtered through a bed of Celite. The filtrate is concentrated to give title A diol.

B. (3α,4α)-7-[Tetrahydro-(1-formyl)-3-thienyl]heptanoic acid, methyl ester

To a solution of 600 mg of title A diol (1.9 mmole) in 5 ml methanol at 25°C is added a solution of 490 mg of Na m-periodate in 1 ml $H_2O$. The mixture is stirred at 25°C for 30 minutes, then extracted with 3—10 ml portions of $CH_2Cl_2$. The organic layer is dried over anhydrous $MgSO_4$ and concentrated to give title aldehyde.

C. [3α,4α]-7-[4-[(Hexyloxy)methyl]tetrahydro-3-thienyl]heptanoic acid

Following the procedure of Example 94 Parts B and C except substituting the above Part B aldehyde for the Example 94 Part A(19) aldehyde, the title acid is obtained.

Example 116

(3α,4α)-7-[4-[(Benzyloxy)methyl]tetrahydro-3-thienyl]heptanoic acid

Following the procedure of Example 115 except substituting benzyl mesylate for hexyl mesylate, the title compound is obtained.

Example 117

(3α,4α)-7-[4-[(Phenyloxy)methyl]tetrahydro-3-thienyl]heptanoic acid

A. (3α,4α)-7-[4-(Hydroxymethyl)tetrahydro-3-thienyl]heptanoic acid, methyl ester

Following the procedure of Example 94 Part B except substituting Example 115, Part B aldehyde for Example 1 Part A aldehyde, the title alcohol is obtained.

B. (3α,4α)-7-[4-[(Phenyloxy)methyl]tetrahydro-3-furanyl]heptanoic acid

Following the procedure of Example 100 except substituting the above title A alcohol for Example 94 Part B alcohol, the title compound is obtained.

Example 118

(3α,4α)-7-[4-[(Cyclohexyloxy)methyl]tetrahydro-3-thienyl]heptanoic acid

Following the procedure of Example 115 except substituting cyclohexyl mesylate for hexyl mesylate, the title compound is obtained.

Example 119

(3α,4α)-7-[4-[(2-Butenyloxy)methyl]tetrahydro-3-thienyl]heptanoic acid

Following the procedure of Example 115 except substituting 2-butenyl-1-mesylate for hexyl mesylate, the title compound is obtained.

Example 120

(3α,4β)-7-[4-[(Benzyloxy)methyl]tetrahydro-3-thienyl]heptanoic acid

Following the procedure of Example 115 except substituting the Example 5 Part A aldehyde for the Example 94 Par A (19) aldehyde and substituting benzyl mesylate for hexyl mesylate, the title compound is obtained.

Example 121

(3α,4β)-7-[4-[(Phenyloxy)methyl]tetrahydro-3-thienyl]heptanoic acid

A. (3α,4β)-7-[4-Formyl-tetrahydro-3-furanyl]heptanoic acid, methyl ester

Following the procedure of Example 98 Part B except substituting Example 115 Part B aldehyde for Example 98 Part A aldehyde, the title aldehyde is obtained.

B. (3α,4β)-7-[4-[(Phenyloxy)methyl]tetrahydro-3-thienyl]heptanoic acid

Following the procedure of Example 117 except substituting the above Part A aldehyde for Example 114 Part B aldehyde in Example 117 Part A, the title acid is obtained.

Example 122

(3α,4β)-7-[4-[(Cyclopropyloxy)methyl]tetrahydro-3-thienyl]heptanoic acid

Following the procedure of Example 115 except substituting the Example 98 Part A aldehyde for the Example 94 Part A (19) aldehyde and substituting cyclopropyl mesylate, the title compound is obtained.

Example 123

(3α,4β)-7-[4-[(Cyclopentylethyloxy)methyl]tetrahydro-3-thienyl]heptanoic acid

Following the procedure of Example 115 except substituting the Example 98 Part A aldehyde for the Example 94 Part A (19) aldehyde and substituting cyclopentylethyl mesylate for hexyl mesylate, the title compound is obtained.

Example 124

(3α,4β)-7-[4-[(3-Hexenyloxy)methyl]tetrahydro-3-thienyl]heptanoic acid

Following the procedure of Example 115 except substituting the Example 98 Part A aldehyde for the Example 94 Part A (19) aldehyde and substituting 3-hexenyl-1-mesylate for hexyl mesylate, the title compound is obtained.

Example 125

(3α,4α)-7-[4-[(Octylthio)methyl]tetrahydro-3-thienyl]heptanoic acid

Following the procedure of Example 115 Parts A and B and Examples 96 and 97 except substituting the Example 115 Part B aldehyde in Examples 96 and 97 for the Example 94 Parts A (16) aldehyde and substituting octylthiol for hexanethiol, the title compound is obtained.

Example 126

(3α,4α)-7-[4-[(Phenylthio)methyl]tetrahydro-3-thienyl]eptanoic acid

Following the procedure of Example 115 Parts A and B and Examples 96 and 97 substituting the Example 115 Part B aldehyde for the Example 94 Part A (19) aldehyde in Examples 96 and 97 and substituting phenylmercaptan for hexanethiol, the title compound is obtained.

Example 127

(3α,4α)-7-[4-[(Benzylthio)methyl]tetrahydro-3-thienyl]heptanoic acid

Following the procedure of Example 115 Parts A and B and Example 96 and 97 substituting the Example 115 Part B aldehyde for the Example 94 Part A (19) aldehyde in Examples 96 and 97 and substituting benzylmercaptan for hexanethiol, the title compound is obtained.

Example 128

(3α,4α)-7-[4-[(Cyclopentylthio)methyl]tetrahydro-3-thienyl]heptanoic acid

Following the procedure of Example 115 Parts A and B and Examples 96 and 97 substituting the Example 115 Part B aldehyde for the Example 94 Part A (19) aldehyde in Examples 96 and 97 and substituting cyclopentylmercaptan for hexanethiol, the title compound is obtained.

Example 129

(3α,4α)-7-[4-[(Cyclohexylmethylthio)methyl]tetrahydro-3-thienyl]heptanoic acid

Following the procedure of Example 115 Parts A and B and Examples 96 and 97 substituting the Example 115 Part B aldehyde for the Example 94 Part (19) aldehyde in Examples 96 and 97 and substituting cyclohexylmethylmercaptan for hexanethiol, the title compound is obtained.

Example 130

(3α,4α)-7-[4-[(2-Octenylthio)methyl]tetrahydro-3-thienyl]heptanoic acid

Following the procedure of Example 115 Parts A and B and Examples 96 and 97 substituting the Example 114 Part B aldehyde for the Example 94 Part A (19) aldehyde in Examples 96 and 97 and substituting 2-octenyl-1-thiol for hexamethiol, the title compound is obtained.

Example 131

[3α(Z),4α]-7-[4-[2-(Hexyloxy)ethyl]tetrahydro-3-thienyl]-5-heptenoic acid

A. [3α(Z),4α]-7-[4-[2-(2-Oxo)ethyl]-tetrahydro-3-thienyl]-5-heptenoic acid, methyl ester

Into a dry 100 ml round bottom 3-necked flask containing a stir bar is added dried 3.27 g (9.54 mmoles) methoxymethyltriphenylphosphonium chloride ($(C_6H_5)_3P^+—CH_2OCH_3Cl^-$) and 30 ml distilled toluene (stored over molecular sieves). The resulting suspension is stirred in an ice-bath, under argon, until cold and then a 1.4 M solution of 5.73 ml (8.01 mmol) of potassium t-amylate in toluene is added dropwise. A bright red solution formed which is stirred at 0°C for an additional 35 minutes. Thereafter, a solution of 900 mg (3.75 mmol) [3α(Z),4α]-7-(4-formyl)tetrahydro-3-thienyl]-5-heptenoic acid, methyl ester (prepared as described in Example 94 Part A (19)) in 10 ml toluene is added by means of a dropping funnel over a 35 minute period with the ice-bath still in place. The reaction is then quenched by addition of 2.3 g (39 mmol) acetic acid in 5 ml ether. The reaction mixture is immediately poured into 200 ml saturated $NH_4Cl$, and extracted with ether (4 × 200 ml). The combined ether phases are washed with NaCl saturated solution, and dried ($MgSO_4$) and concentrated to yield an oil in a white crystalline solid (phosphine oxide). The white solid is triturated with EtOAc and the mother liquor is purified by chromatography on an LPS—1 silica column to obtain the enol-ether. The enol-ether is dissolved in 20 ml of THF and is then treated with 10 ml of a 20% aqueous trifluoroacetic acid solution. After 1 hour, trifluoroacetic acid is quenched by addition of solid $NaHCO_3$. The reaction mixture is extracted several times with methylene chloride. The methylene chloride extract is dried over anhydrous magnesium sulfate and concentrated under reduced pressure. The crude residue is chromatographed on a silica gel column to obtain the desired title A aldehyde.

B. [3α(Z),4α]-7-[[4-[(2-Hydroxyethyl)]tetrahydro-3-thienyl]-5-heptenoic acid, methyl ester

The aldehyde (762 mg, 3 mmol) from part A in methanol (50 ml) is treated with $NaBH_4$ (0.11 g, 3 mmol) in an argon atmosphere at 0°C. After stirring at 0°C for 1 hour, the reaction is quenched by addition of 2N HCl (to pH 2). The methanol is removed *in vacuo* and the reaction mixture is taken up in ether. The ether solution is washed with saturated $KHCO_3$, saturated NaCl and dried ($MgSO_4$). The ether is evaporated to yield the title B compound.

C. [3α(Z),4α]-7-[[4-[2-(Hexyloxy)ethyl]tetrahydro-3-thienyl]-5-heptenoic acid

Following the procedure of Example 94, Part C and Example 98 except substituting the above part B alcohol for the Example 94 Part B alcohol used in Example 94 Part C, the title compound is obtained.

Example 132

[3α(Z),4α]-7-[4-[2-(Hexylthio)ethyl]tetrahydro-3-thienyl]-5-heptenoic acid

Following the procedure of Examples 96 and 97 except substituting [3α(Z),4α]-7-[4-[2-(2-oxo)ethyl]-tetrahydro-3-thienyl]-5-heptenoic acid, methyl ester (prepared as described in Example 131 Part A) for the Example 94 Part B alcohol used in Example 96 Part A, the title compound is obtained.

Example 133

[3α(Z),4α]-7-[4-[2-(Benzyloxy)ethyl]tetrahydro-3-thienyl]-5-heptenoic acid

Following the procedure of Example 131 except substituting benzyl mesylate for hexyl mesylate, the title compound is obtained.

Example 134

[3α(Z),4α]-7-[4-[2-(Phenyloxy)ethyl]tetrahydro-3-thienyl]-5-heptenoic acid

Following the procedure of Example 100 except substituting Example 131 Part B alcohol for Example 94 Part B alcohol, the title compound is obtained.

Example 135

[3α(Z),4α]-7-[4-[2-(Butenyloxy)ethyl]tetrahydro-3-thienyl]-5-heptenoic acid

Following the procedure of Example 131 except substituting 1-butenyl mesylate for hexyl mesylate, the title compound is obtained.

Example 136

[3α(Z),4α]-7-[4-[2-(Cyclohexyloxy)ethyl]tetrahydro-3-thienyl]-5-heptenoic acid

Following the procedure of Example 131 except substituting cyclohexyl mesylate for hexyl mesylate, the title compound is obtained.

Example 137

[3α(Z),4α]-7-[4-[2-(Propyloxy)ethyl]tetrahydro-3-thienyl]-5-heptenoic acid

Following the procedure of Example 131 except substituting n-propyl mesylate for hexyl mesylate, the title compound is obtained.

Example 138

[3α(Z),4α]-7-[4-[2-(Cyclopentylmethyloxy)ethyl]tetrahydro-3-thienyl]-5-heptenoic acid

Following the procedure of Example 131 except substituting cyclopentylmethyl mesylate for hexyl mesylate, the title compound is obtained.

Example 139

[3α(Z),4α]-7-[4-[2-(Pentyloxy)ethyl]tetrahydro-3-thienyl]-5-heptenoic acid

Following the procedure of Example 132 except substituting 1-pentanethiol for 1-hexanethiol, the title compound is obtained.

Example 140

[3α(Z),4α]-7-[4-[2-(Benzylthio)ethyl]tetrahydro-3-thienyl]-5-heptenoic acid

Following the procedure of Example 132 except substituting benzylmercaptan for 1-hexanethiol, the title compound is obtained.

Example 141

[3α(Z),4α]-7-[4-[2-(Phenylthio)ethyl]tetrahydro-3-thienyl]-5-heptenoic acid

Following the procedure of Example 132 except substituting phenylmercaptan for 1-hexanethiol, the title compound is obtained.

Example 142

[3α(Z),4α]-7-[4-[2-(Cyclohexylthio)ethyl]tetrahydro-3-thienyl]-5-heptenoic acid

Following the procedure of Example 132 except substituting cyclohexylmercaptan for 1-hexanethiol, the title compound is obtained.

Example 143

[3α(Z),4α]-7-[4-[2-(Cyclohexylmethylthio)ethyl]tetrahydro-3-thienyl]-5-heptenoic acid

Following the procedure of Example 132 except substituting cyclohexylmethylmercaptan for 1-hexanethiol, the title compound is obtained.

Example 144

[3α(Z),4α]-7-[4-[2-(1-Propenylthio)ethyl]tetrahydro-3-thienyl]-5-heptenoic acid

Following the procedure of Example 132 except substituting 1-(1-propenyl)thiol for 1-hexanethiol, the title compound is obtained.

Example 145

[3α,4α]-7-[4-[2-(Hexyloxy)ethyl]tetrahydro-3-thienyl]heptenoic acid

Following the procedure of Example 131 except substituting the Example 115 Part B aldehyde for the Example 94 Part A (19) aldehyde used in Example 131 Part A, the title compound is obtained.

Example 146

[3α,4α]-7-[4-[2-(Cyclopentylethyloxy)ethyl]tetrahydro-3-thienyl]heptanoic acid

Following the procedure of Example 131 except substituting the Example 115 Part B aldehyde for the Example 94 Part A (19) aldehyde used in Example 131 Part A and substituting cyclopentylethyl mesylate for hexyl mesylate, the title compound is obtained.

Example 147

[3α,4α]-7-[4-[2-(Benzyloxy)ethyltetrahydro-3-thienyl]heptanoic acid

Following the procedure of Example 131 except substituting the Example 115 Part B aldehyde for the Example 94 Part A (19) aldehyde used in Example 131 Part A and substituting benzyl mesylate for hexyl mesylate, the title compound is obtained.

Example 148

[3α,4α]-7-[4-[2-(Phenyloxy)ethyl]tetrahydro-3-thienyl]-5-heptenoic acid

Following the procedure of Examples 131 and 100 except substituting the Example 115 Part B aldehyde for the Example 94 Part A (19) aldehyde used in Example 131 Part A and substituting the resulting (3α,4α)-7-[4-[2-(hydroxyethyl)]tetrahydro-3-thienyl]heptenoic acid, methyl ester for Example 94 Part B alcohol in Example 100 Part (a), the title compound is obtained.

Example 149

[3α,4α]-7-[4-[2-(Cyclopentyloxy)ethyl]tetrahydro-3-thienyl]heptenoic acid

Following the procedure of Example 131 except substituting the Example 115 Part B aldehyde for the Example 94 Part A (19) aldehyde used in Example 131 Part A and substituting cyclopentyl mesylate for hexyl mesylate, the title compound is obtained.

Example 150

[3α,4α]-7-[4-[2-(3-Hexenyloxy)ethyl]tetrahydro-3-thienyl]heptenoic acid

Following the procedure of Example 131 except substituting the Example 115 Part B aldehyde for the Example 94 Part A (19) aldehyde used in Example 131 Part A and substituting 3-hexenyl mesylate for hexyl mesylate, the title compound is obtained.

Example 151

[3α,4α]-7-[4-[2-(Cyclopropylmethylthio)ethyl]tetrahydro-3-thienyl]heptenoic acid

Following the procedure of Example 132 except substituting the Example 115 Part B aldehyde for the Example 131 Part A aldehyde and substituting cyclopropylmethyl mercaptan for 1-hexanethiol, the title compound is obtained.

Example 152

[3α,4α]-7-[4-[2-(Benzylthio)ethyl]tetrahydro-3-thienyl]heptenoic acid

Following the procedure of Example 132 except substituting the Example 115 Part B aldehyde for the Example 131 Part A aldehyde and substituting benzylmercaptan for 1-hexanethiol, the title compound is obtained.

Example 153

[3α,4α]-7-[4-[2-(Phenylthio)ethyl]tetrahydro-3-thienyl]heptenoic acid

Following the procedure of Example 132 except substituting the Example 115 Part B aldehyde for the Example 131 Part A aldehyde and substituting phenylmercaptan for 1-hexanethiol, the title compound is obtained.

Example 154

[3α,4α]-7-[4-[2-(Cyclohexylthio)ethyl]tetrahydro-3-thienyl]heptenoic acid

Following the procedure of Example 132 except substituting the Example 115 Part B aldehyde for the Example 131 Part A aldehyde and substituting cyclohexylmercaptan for 1-hexanethiol, the title compound is obtained.

Example 155

[3α,4α]-7-[4-[2-(2-Heptenylthio)ethyl]tetrahydro-3-thienyl]heptenoic acid

Following the procedure of Example 132 except substituting the Example 115 Part B aldehyde for the Example 131 Part A aldehyde and substituting 1-(2-heptenyl)thiol for 1-hexanethiol, the title compound is obtained.

Example 156

[3α(Z),4α]-7-[4-[4-(Hexyloxy)butyl]tetrahydro-3-thienyl]-5-heptenoic acid

A. [3α(Z),4α]-7-[4-[3-[3-Oxo)propyl]tetrahydro-3-thienyl]-5-heptenoic acid, methyl ester

Following the procedure of Example 131 Part A except substituting [3α(Z),4α]-7-[4-[2-(2-oxo)ethyl]-tetrahydro-3-thienyl]-5-heptenoic acid, methyl ester for [3α(Z),4α]-7-[(4-formyl)tetrahydro-3-thienyl]-5-heptenoic acid, methyl ester, the title A compound is obtained.

B. [3α(Z),4α]-7-[4-[4-Oxo)butyl]tetrahydro-3-thienyl]-5-heptenoic acid, methyl ester

Following the procedure of Example 131 Part A except substituting the aldehyde from Part A above for [3α(Z),4α]-7-[4-[2-(2-oxo)ethyl]tetrahydro-3-thienyl]-5-heptenoic acid, methyl ester, the title B aldehyde is obtained.

C. [3α(Z),4α]-7-[4-(4-Hydroxybutyl)]tetrahydro-3-thienyl]-5-heptenoic acid, methyl ester

Following the procedure of Example 131 Part B except substituting the title B aldehyde for [3α(Z),4α]-7-[4-[2-(2-oxo)ethyl]tetrahydro-3-thienyl]-5-heptenoic acid, methyl ester, the title C compound is obtained.

D. [3α(Z),4α]-7-[4-[4-Hexyloxy)butyl-tetrahydro-3-thienyl]-5-heptenoic acid

Following the procedure of Example 94 except substituting the above part C alcohol for the alcohol used in Example 94 Part C, the title compound is obtained.

Example 157

[3α(Z),4α]-7-[4-[4-(Benzyloxy)butyl]tetrahydro-3-thienyl]-5-heptenoic acid

Following the procedure of Examples 156 and 131 except substituting the Example 156 Part C alcohol for the Example 131 Part A alcohol and substituting benzyl mesylate for hexyl mesylate, the title compound is obtained.

Example 158

[3α(Z),4α]-7-[4-[4-(Cyclohexylthio)butyl]tetrahydro-3-thienyl]-5-heptenoic acid

Following the procedure of Examples 156, 131 and 132 except substituting the Example 156 Part C alcohol for the Example 131 Part B alcohol and substituting cyclohexylmercaptan for 1-hexanethiol, the title compound is obtained.

Example 159

[3α(Z),4α]-7-[4-[(Hexylsulfinyl)methyl]tetrahydro-3-thienyl]-5-heptenoic acid and [3α(Z),4α]-7-[4-(Hexylsulfonyl)methyl]tetrahydro-3-thienyl]-5-heptenoic acid

To a solution of 562 mg (1.72 mmol) of [3α(Z),4α]-7-[4-[(hexylthio)methyl]tetrahydro-3-thienyl]-5-heptenoic acid, methyl ester (prepared as described in Example 97) in 6.78 ml of methanol at 0°C is added dropwise over 4 minutes 8.37 ml of 0.5M aqueous sodium periodate solution. Tetrahydrofuran (2 ml) is then added and the resulting reaction mixture is stirred at room temperature for 15 hours. A white precipitate is removed by filtration and washed with ether (3 × 50 ml). The filtrate is washed with 60 ml of saturated aqueous $NaHCO_3$ solution and dried over anhydrous magnesium sulfate. Concentration *in vacuo* affords 539 mg of an oily crude product. This is chromatographed on 54.16 g of silica gel 60 using 0.5—1.0% $CH_3OH$ to give the title compounds.

Example 160

[3α(Z),4α]-7-[4-[(Hexylsulfonyl)methyl]tetrahydro-3-thienyl]-5-heptenoic acid

To a stirred solution of 143 mg (0.4 mmol) of the Example 159 sulfonyl compound in 20.3 ml of THF and 3.09 ml of $H_2O$ under argon is added 3.90 ml of 1N aqueous lithium hydroxide solution. This mixture is purged with argon vigorously for 10 minutes and stirred at room temperature for 6 hours. The reaction mixture is acidified to pH 4 by addition of 1N aqueous HCl solution and poured into 30 ml of saturated NaCl solution. The resulting solution is saturated with solid NaCl and extracted with EtOAc (4 × 50 ml). The combined EtOAc extracts are dried ($MgSO_4$), filtered and concentrated *in vacuo* to give 140 mg of crude acid which is purified by flash chromatography.

Example 161

[3α(Z),4α]-7-[4-[(Hexylsulfinyl)methyl]tetrahydro-3-thienyl]-5-heptenoic acid

To a stirred solution of 120 mg (0.35 mmol) of Example 159 ester and sulfinyl ester in 27.0 ml of THF and 4.11 ml of $H_2O$ under argon is added 5.19 ml of 1N aqueous lithium hydroxide solution. This mixture is purged with argon vigorously for 10 minutes and stirred at room temperature for 6 hours. The reaction mixture is acidified to pH 4 by addition of 1N aqueous HCl solution and poured into 50 ml of saturated NaCl solution. The resulting solution is saturated with solid NaCl and extracted with EtOAc (4 × 100 ml). The combined EtOAc extracts are dried ($MgSO_4$), filtered and concentrated *in vacuo* to give crude acid which is purified by flash chromatography.

Example 162

[3α(Z),4β]-7-[4-[2-(Hexyloxymethyl)]tetrahydro-3-thienyl]-2,5-heptadienoic acid

A. [3α(Z),4β]-7-[4-[2-(Hexyloxymethyl)]tetrahydro-3-thienyl]-2-selenophenyl-5-heptenoic acid, methyl ester

To a solution of 308 μl of diisopropylamine (2.2 mmole) in 5 ml of dry THF, cooled at −78°C, is added dropwise 1.25 ml of a 1.6 *M* solution of n-butyllithium in hexane. After 30 minutes at −78°C, a solution of 356 mg of [3α(Z),4β]-7-[4-[(hexyloxy)methyl]tetrahydro-3-thienyl]-5-heptenoic acid, hexyl ester, prepared as described in Example 5, (1 mmole) in 2 ml of dry THF is added dropwise. The reaction mixture is stirred for 30 minutes, whereupon a solution of 625 mg of diphenyldiselenide (2 mmole) in 2 ml of dry THF is added. The yellow color of diselenide disappears immediately upon its addition, initially. The yellow solution is stirred at −78°C for 30 minutes, whereupon the cooling bath was removed. The reaction mixture is then quenched by addition of aqueous ammonium chloride solution. It is then diluted with water and the organic layer is separated. The aqueous layer is extracted with ether. The combined organic extract is dried over anhydrous magnesium sulfate and concentrated *in vacuo*. Purification by chromatography on a silica gel column and eluting with 5—25% ethyl acetate in hexane gives 600 mg of title α-selenophenyl ester (90% yield).

B. [3α(Z),4β]-7-[4-[2-(Hexyloxymethyl)]tetrahydro-3-thienyl]-2-selenophenyl-5-heptenoic acid

A solution of 600 mg of title A α-selenophenyl esters in 10 ml of distilled THF is treated with 5 ml of a 1 *N* aqueous lithium hydroxide solution. After stirring at room temperature for 2 days, the reaction mixture is acidified with 1 *N* aqueous hydrochloric acid solution and extracted with methylene chloride. The methylene chloride extract is dried over anhydrous magnesium sulfate and concentrated *in vacuo* to yield 560 mg of title acid.

C. [3α(Z),4β]-7-[4-[2-(Hexyloxymethyl)]tetrahydro-3-thienyl]-2,5-heptadienoic acid

A solution of 560 mg of title B acid (0.86 mmole) in 10 ml of distilled THF is treated with 500 ml of a 30% aqueous hydrogen peroxide solution at 0—5°C. After a few minutes, the cooling bath is removed and the reaction mixture is stirred at room temperature for 1 hour. It is then diluted with methylene chloride and washed thoroughly with water. The organic layer is dried over anhydrous $MgSO_4$ and concentrated under reduced pressure. The crude residue is chromatographed on a CC—7 silica gel column and eluted with 20—60% ethyl acetate in hexane to obtain the title α,β-unsaturated acid.

Example 163

[3α(Z),4α]-7-[4-[2-(Hexylthio)methyl)]tetrahydro-3-thienyl]-2,5-heptadienoic acid

Following the procedure of Example 162 except substituting the Example 96 compound for the Example 98 compound in Example 162 Part A, the title compound is obtained.

Example 164

[3α(Z),4α]-7-[4-[2-(Phenyloxy)methyl)]tetrahydro-3-thienyl]-2,5-heptadienoic acid

Following the procedure of Example 162 except substituting the ester compound prepared in Example 100 for the Example 98 compound in Example 162 Part A, the title compound is obtained.

Example 165

[3α(Z),4α]-7-[4-[2-(Benzoyloxy)methyl]tetrahydro-3-thienyl]-2,5-heptadienoic acid

Following the procedure of Example 162 except substituting the ester compound prepared in Example 101 for the Example 98 compound in Example 162 Part A, the title compound is obtained.

Example 166

[3α(Z),4α]-7-[4-[(2-Pentenyloxy)methyl]tetrahydro-3-thienyl]-2,5-heptadienoic acid

Following the procedure of Example 162 except substituting the ester compound prepared in Example 103 for the Example 98 compound in Example 162 Part A, the title compound is obtained.

Example 167

[3α(Z),4α]-7-[4-[(Heptyloxy)methyl)]tetrahydro-3-thienyl]-2,5-heptadienoic acid

Following the procedure of Example 162 except substituting the ester compound prepared in Example 105 for the Example 98 compound in Example 162 Part A, the title compound is obtained.

Example 168

[3α(Z),4α]-7-[4-[(Cyclopentyloxy)methyl]tetrahydro-3-thienyl]-2,5-heptadienoic acid

Following the procedure of Example 162 except substituting the ester compound prepared in Example 107 for the Example 98 compound in Example 117 Part A, the title compound is obtained.

Example 169

[3α(Z),4β]-7-[4-[(Cyclohexyloxy)methyl]tetrahydro-3-thienyl]-2,5-heptadienoic acid

Following the procedure of Example 162 except substituting the ester compound prepared in Example 108 for the Example 98 compound in Example 162 Part A, the title compound is obtained.

Example 170

[3α(Z),4β]-7-[4-[(3-Butenyloxy)methyl]tetrahydro-3-thienyl]-2,5-heptadienoic acid

Following the procedure of Example 162 except substituting the ester compound prepared in Example 109 for the Example 98 compound in Example 161 Part A, the title compound is obtained.

Example 171

[3α(Z),4α]-7-[4-[(2-Hexenylthio)methyl]tetrahydro-3-thienyl]-2,5-heptadienoic acid

Following the procedure of Example 162 except substituting the ester compound prepared in Example 110 for the Example 98 compound in Example 162 Part A, the title compound is obtained.

Example 172

[3α,4α]-7-[4-[(Hexyloxy)methyl)]tetrahydro-3-thienyl]-2,5-heptadienoic acid

Following the procedure of Example 162 except substituting the ester compound prepared in Example 115 for the Example 98 compound in Example 162 Part A, the title compound is obtained.

Example 173

[3α,4α]-7-[4-[(Octylthio)methyl)]tetrahydro-3-thienyl]-2-heptenoic acid

Following the procedure of Example 162 except substituting the ester compound prepared in Example 125 for the Example 98 compound in Example 162 Part A, the title compound is obtained.

Example 174

[3α(Z),4α]-7-[4-[2-(Hexyloxy)ethyl)]tetrahydro-3-thienyl]-2,5-heptadienoic acid

Following the procedure of Example 162 except substituting the ester compound prepared in Example 131 for the Example 98 compound in Example 162 Part A, the title compound is obtained.

Example 175

[3α(Z),4α]-7-[4-[2-(Hexylthio)ethyl)]tetrahydro-3-thienyl]-2,5-heptadienoic acid

Following the procedure of Example 162 except substituting the ester compound prepared in Example 132 for the Example 98 compound in Example 162 Part A, the title compound is obtained.

Example 176

[3α,4α]-7-[4-[2-(Hexyloxy)ethyl)]tetrahydro-3-thienyl]-2-heptenoic acid

Following the procedure of Example 162 except substituting the ester compound prepared in Example 145 for the Example 94 compound in Example 162 Part A, the title compound is obtained.

Example 177

[3α,4α]-7-[4-[2-(Cyclohexylthio)ethyl)]tetrahydro-3-thienyl]-2-heptenoic acid

Following the procedure of Example 162 except substituting the ester compound prepared in Example 154 for the Example 94 compound in Example 162 Part A, the title compound is obtained.

Examples 178 to 187

Following the procedure as outlined in the specification and described in the working Examples, the following additional compounds may be prepared.

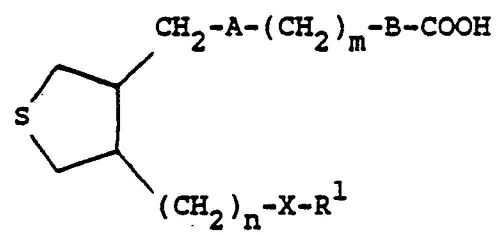

| Ex. No. | A | m | B | $(CH_2)_n$ | $R^1$ | X |
|---|---|---|---|---|---|---|
| 178. | CH=CH | 1 | CH=CH | $CH_2$ | $CH_3CH=CH$ | S |
| 179. | $(CH_2)_2$ | 2 | CH=CH | $(CH_2)_2$ | $CH_3CH_2CH=CHCH_2$- | O |
| 180. | --- | 3 | CH=CH | $(CH_2)_3$ | 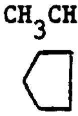 | S<br>‖<br>O |
| 181. | CH=CH | 4 | --- | $-CHCH_2-$<br>$CH_3$ | 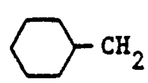 | O<br>‖<br>S<br>‖<br>O |
| 182. | $(CH_2)_2$ | 5 | CH=CH | $(CH_2)_4$ | $C_6H_5$ | S |
| 183. | CH=CH | 6 | -- | $(CH_2)_5$ | $C_6H_5(CH_2)_2$ | O |
| 184. | $CH-CH_2$<br>$CH_3$ | 7 | --- | $(CH_2)_6$ | $C_6H_5CH_2$ | S |
| 185. | $CH_2CH$ | 8 | CH=CH | $(CH_2)_7$ | $C_6H_{13}$ | S<br>‖<br>O |
| 186. | $(CH_2)_2$ | 6 | CH=CH | $(CH_2)_8$ | $C_7H_{15}$ | O<br>‖<br>S<br>‖<br>O |
| 187. | --- | 2 | --- | $(CH_2)_2$ | $C_2H_5$ | O |

**Claims**

1. A compound of the structure

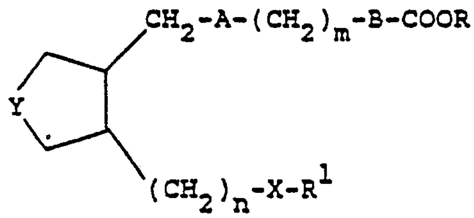

including all stereoisomers thereof, wherein A is $(CH_2)_2$, CH=CH or a single bond; B is a single bond or CH=CH; m is 1 to 8; n is 1 to 4; X is O or

S
||
$(O)_{n'}$

wherein n' is 0, 1 or 2; R is H, lower alkyl or alkali metal; and $R^1$ is lower alkyl, aryl, arylalkyl, cycloalkyl, cycloalkylalkyl or lower alkenyl and Y is sulfur or oxygen, the lower alkyl and alkyl groups having 1 to 12 carbon atoms and being optionally substituted by F, Br, Cl, I, $CF_3$, an alkoxy substituent, an aryl substituent, an alkyl-aryl substituent, a haloaryl substituent, a cycloalkyl substituent or an alkylcycloalkyl substituent, the lower alkenyl groups having 2 to 12 carbon atoms, the aryl groups having 6 to 10 carbon atoms and being optionally substituted by lower alkyl, halogen or lower alkoxy when aryl is phenyl or naphthyl, the cycloalkyl groups having 3 to 12 carbon atoms and the $(CH_2)_2$, $(CH_2)_m$ and $(CH_2)_n$ groups being optionally substituted by one or more alkyl groups.

2. The compound as defined in Claim 1 wherein Y is oxygen.

3. The compound as defined in Claim 1 wherein Y is sulfur.

4. The compound as defined in Claims 1, 2 or 3 wherein A is CH=CH and B is a single bond.

5. The compound as defined in Claims 1, 2 or 3 wherein X is O or S.

6. The compound as defined in Claims 1, 2 or 3 wherein m is 3 to 5 and n is 1.

7. The compound as defined in Claims 1, 2 or 3 wherein $R^1$ is H.

8. The compound as defined in Claims 1, 2 or 3 wherein B is a single bond, n is 1, A is CH=CH, X is O or S, n is 1, m is 3 to 5, R is H and $R^1$ is lower alkyl.

9. The compound as defined in Claims 1, 2 or 3 wherein $R^1$ is butyl, pentyl, hexyl or heptyl, including all isomers thereof.

10. The compound as defined in Claim 1 having the name [3α(Z),4α]-7-[4-[(hexyloxy)methyl]tetrahydro-3-furanyl]-5-heptenoic acid, including all stereoisomers thereof.

11. The compound as defined in Claim 1 having the name [3α(Z),4β]-7-[4-[(hexyloxy)methyl]tetrahydro-3-furanyl]-5-heptenoic acid or the hexyl ester thereof, including all stereoisomers thereof.

12. The compound as defined in Claim 1 having the name [3α(Z),4α]-7-[4-[(hexylthio)methyl]tetrahydro-3-furanyl]-5-heptenoic acid or its methyl ester, including all stereoisomers thereof.

13. The compound as defined in Claim 1 having the name [3α(Z),4α]-7-[4-[(hexyloxy)methyl]tetrahydro-3-thienyl]-5-heptenoic acid or the hexyl ester thereof, including all stereoisomers thereof.

14. The compound as defined in Claim 1 having the name [3α(Z),4α]-7-[4-[(hexylthio)methyl]tetrahydro-3-thienyl]-5-heptenoic acid or its methyl ester, including all stereoisomers thereof.

**Patentansprüche**

1. Eine Verbindung der Struktur

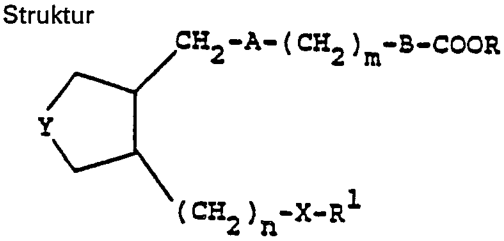

einschließlich aller Stereoisomeren davon, wobei A $(CH_2)_2$, CH=CH oder eine Einfachbindung bedeutet; B eine Einfachbindung oder CH=CH darstellt, m 1 bis 8 ist, n 1 bis 4 ist, X O oder

S
||
$(O)_{n'}$

wobei n' 0, 1 oder 2 ist, R H, Niederalkyl oder ein Alkalimetall darstellt, und $R^1$ Niederalkyl, Aryl, Arylalkyl, Cycloalkyl, Cycloalkylalkyl oder Niederalkenyl bedeutet und Y Schwefel oder Sauerstoff ist, wobei die Niederalkyl- und Alkylreste 1 bis 12 Kohlenstoffatome aufweisen und gegebenenfalls durch F, Br, Cl, I, $CF_3$, einen Alkoxysubstituenten, einen Arylsubstituenten, einen Alkylarylsubstituenten, einen Halogenarylsubstituenten, einen Cycloalkylsubstituenten oder einen Alkylcycloalkylsubstituenten substituiert sind, die Niederalkinylreste 2 bis 12 Kohlenstoffatome aufweisen, die Arylreste 6 bis 10 Kohlenstoffatome aufweisen und gegebenenfalls durch Niederalkyl, Halogen oder Niederalkoxy substituiert sind, wenn Aryl Phenyl oder Naphthyl darstellt, die Cycloalkylreste 3 bis 12 Kohlenstoffatome aufweisen und die Reste $(CH_2)_2$, $(CH_2)_m$ und $(CH_2)_n$ gegebenenfalls durch einen oder mehrere Alkylreste substituiert sind.

2. Verbindung nach Anspruch 1, in der Y Sauerstoff ist.

3. Verbindung nach Anspruch 1, in der Y Schwefel ist.

4. Verbindung nach Anspruch 1, 2 oder 3, in der A CH=CH bedeutet und B eine Einfachbindung ist.

5. Verbindung nach Anspruch 1, 2 oder 3, in der X O oder S ist.

6. Verbindung nach Anspruch 1, 2 oder 3, in der m 3 bis 5 ist und n 1 ist.

7. Verbindung nach Anspruch 1, 2 oder 3, in der $R^1$ H ist.

8. Verbindung nach Anspruch 1, 2 oder 3, in der B eine Einfachbindung ist, n 1 ist, A CH=CH bedeutet, X O oder S darstellt, n 1 ist, m 3 bis 5 ist, R H bedeutet und $R^1$ Niederalkyl darstellt.

9. Verbindung nach Anspruch 1, 2 oder 3, in der $R^1$ Butyl, Pentyl, Hexyl oder Heptyl einschließlich aller Isomeren davon bedeuten.

10. Verbindung nach Anspruch 1 mit dem Namen [3α(Z),4α]-7-[4-[(Hexyloxy)-methyl]-tetrahydro-3-furanyl]-5-heptensäure einschließlich aller Stereoisomeren davon.

11. Verbindung nach Anspruch 1 mit dem Namen [3α(Z),4β]-7-[4-[(Hexyloxy)-methyl]-tetrahydro-3-furanyl]-5-heptensäure oder der Hexylester davon einschließlich aller Stereoisomeren davon.

12. Verbindung nach Anspruch 1 mit dem Namen [3α(Z),4α]-7-[4-[(Hexylthio)-methyl]-tetrahydro-3-furanyl]-5-heptensäure oder ihr Methylester einschließlich aller Stereoisomeren davon.

13. Verbindung nach Anspruch 1 mit dem Namen [3α(Z),4α]-7-[4-[(Hexyloxy)-methyl]-tetrahydro-3-thienyl]-5-heptensäure oder der Hexylester davon einschließlich aller Stereoisomeren davon.

14. Verbindung nach Anspruch 1 mit dem Namen [3α(Z),4α]-7-[4-[(Hexylthio)-methyl]-tetrahydro-3-thienyl]-5-heptensäure oder ihre Methylester einschließlich aller Stereoisomeren davon.

**Revendications**

1. Composé de formule

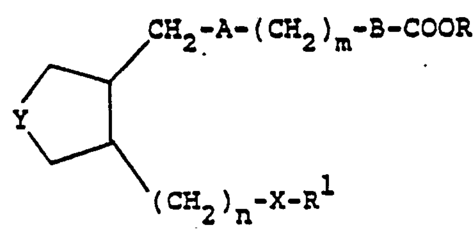

y compris tous ses stéréo-isomères, formule dans laquelle A est $(CH_2)_2$, CH=CH ou une liaison simple; B est une liaison simple ou CH=CH; m est un nombre de 1 à 8; n est un nombre de 1 à 4; X est O ou

$$\underset{(O)_{n'}}{\overset{}{\underset{\|}{S}}}$$

où n' est égal à 0, 1 ou 2; R est un atome d'hydrogène, un groupe alkyle inférieur ou un atome de métal alcalin; et $R^1$ est un groupe alkyle inférieur, aryle, arylalkyle, cycloalkyle, cycloalkylalkyle ou alcényle inférieur et Y est un atome de soufre ou d'oxygène, les groupes alkyle inférieur et alkyle ayant de 1 à 12 atomes de carbone et étant éventuellement substitués par F, Br, Cl, I, $CF_3$, un substituant alcoxy, un substituant aryle, un substituant alkyl-aryle, un substituant haloaryle, un substituant cycloalkyle ou un substituant alkylcycloalkyle, les groupes alcényle inférieur ayant de 2 à 12 atomes de carbone, les groupes aryle ayant de 6 à 10 atomes de carbone et étant éventuellement substitués par alkyle inférieur, halogène ou alcoxy inférieur quand le groupe aryle est un groupe phényle ou naphtyle, les groupes cycloalkyle ayant de 3 à 12 atomes de carbone et les groupes $(CH_2)_2$, $(CH_2)_m$ et $(CH_2)_n$ étant facultativement substitués par un ou plusieurs groupes alkyle.

2. Composé selon la revendication 1, dans lequel Y est l'oxygène.

3. Composé selon la revendication 1, dans lequel Y est le soufre.

4. Composé selon les revendications 1, 2 ou 3, dans lequel A est CH=CH et B est une liaison simple.

5. Composé selon les revendications 1, 2 ou 3, dans lequel X est O ou S.

6. Composé selon les revendications 1, 2 ou 3, dans lequel m est un nombre de 3 à 5 et n est égal à 1.

7. Composé selon les revendications 1, 2 ou 3, dans lequel $R^1$ est l'hydrogène.

8. Composé selon les revendications 1, 2 ou 3, dans lequel B est une liaison simple, n est égal à 1, A est CH=CH, X est O ou S, n est égal à 1, m est un nombre de 3 à 5, R est l'hydrogène et $R^1$ est un groupe alkyle inférieur.

9. Composé selon les revendications 1, 2 ou 3, dans lequel $R^1$ est un groupe butyle, pentyle, hexyle ou heptyle, y compris tous ses isomères.

10. Composé selon la revendication 1, qui est l'acide [3α(Z),4α]-7-[4-[(hexyloxy)-méthyl]tétrahydro-3-furanyl]-5-hepténoïque, y compris tous ses stéréo-isomères.

11. Composé selon la revendication 1, qui est l'acide [3α(Z),4β]-7-[4-[(hexyloxy)méthyl]tétrahydro-3-furanyl]-5-hepténoïque ou son ester hexylique, y compris tous leurs stéréo-isomères.

12. Composé selon la revendication 1, qui est l'acide [3α(Z),4α]-7-[4-[(hexylthio)méthyl]tétrahydro-3-furanyl]-5-hepténoïque ou son ester méthylique, y compris tous leurs stéréo-isomères.

13. Composé selon la revendication 1, qui est l'acide [3α(Z),4α]-7-[4-[(hexyloxy)méthyl]tétrahydro-3-thienyl]-5-hepténoïque ou son ester hexylique, y compris tous leurs stéréo-isomères.

14. Composé selon la revendication 1, qui est l'acide [3α(Z),4α]-7-[4-[(hexylthio)méthyl]tétrahydro-3-thienyl]-5-hepténoïque ou son ester méthylique, y compris tous leurs stéréo-isomères.